(19) Europäisches Patentamt
European Patent Office
Office européen des brevets

(11) **EP 3 622 967 A1**

(12) **EUROPEAN PATENT APPLICATION**
published in accordance with Art. 153(4) EPC

(43) Date of publication:
**18.03.2020 Bulletin 2020/12**

(51) Int Cl.:
*A61K 47/28* (2006.01)  *A61K 9/127* (2006.01)
*A61K 47/14* (2017.01)  *A61K 47/22* (2006.01)
*A61K 47/24* (2006.01)  *A61K 47/26* (2006.01)
*A61K 47/44* (2017.01)

(21) Application number: 18798656.7

(22) Date of filing: 09.05.2018

(86) International application number:
**PCT/JP2018/017982**

(87) International publication number:
**WO 2018/207841 (15.11.2018 Gazette 2018/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.05.2017 JP 2017094952**

(71) Applicant: **Terumo Kabushiki Kaisha
Tokyo 151-0072 (JP)**

(72) Inventor: **SAKAGUCHI, Naoki
Kanagawa 259-0151 (JP)**

(74) Representative: **Casalonga
Casalonga & Partners
Bayerstraße 71/73
80335 München (DE)**

(54) **COMPOSITE BODY, pH-SENSITIVE COMPOSITION CONTAINING COMPOSITE BODY, AND METHOD FOR PRODUCING COMPOSITE BODY**

(57)    According to the present invention, there is provided a complex in which a carrier and a physiologically active substance are integrated, the complex being conveniently obtainable, having excellent efficiency for the integration of a physiologically active substance, and having a satisfactory membrane disruptive function promoting effect. The complex according to the present invention is formed by supporting a physiologically active substance on an aggregate of a pH sensitive carrier, wherein the aggregate is formed by aggregating under acidic conditions.

FIG. 2(A)

EP 3 622 967 A1

FIG. 2(B)

**Description**

**TECHNICAL FIELD**

The present invention relates to a complex useful as a DDS (Drug Delivery System), a pH sensitive composition including the complex, and a method for producing the complex.

**BACKGROUND ART**

In recent years, research on DDS carriers (carriers) for delivering physiologically active substances is actively in progress. Carriers having sensitivity to weakly acidic pH are expected to actualize delivery to a site where the pH has been decreased in vivo, such as a tumor or an inflammation site, or delivery to the cytosol of cells through endosome escape.

Particularly, since delivery to the cytosol can be utilized in a variety of fields, realization thereof is highly desired. For example, delivery of nucleic acid to the cytosol enables gene therapy, and delivery of an antigen to the cytosol is a technology that serves as a key to the induction of antigen-specific CTLs (Cytotoxic T Lymphocytes), which is useful for immunotherapy or the like, or to the induction of antigen-specific Treg (regulatory T cell), which is useful for the treatment of allergies and the like. Furthermore, application thereof to novel drugs that are purported to utilize the inside of the cell as the drug target has also been investigated.

As a pH sensitive carrier that realizes the above-described delivery, a pH sensitive carrier containing a particular amphiphilic substance and a particular pH sensitive compound is disclosed in WO 2013/180253 A (corresponding to US 2013/323320 A, US 2016/151497 A, and US 2018/104343 A). This pH sensitive carrier is considered to have an ability to destroy the biological membrane in a pH range of from neutral pH to pH 4.0, which is the attained pH of endosomes and lysosomes, and to deliver a model physiologically active substance to the cytosol of cells. Furthermore, in WO 2015/079952 A (corresponding to US 2016/271246 A) and WO 2016/194685 A (corresponding to EP 3305320 A), it is disclosed that when such a pH sensitive carrier is used together with Lipid A, which is a substance that stimulates natural immunity, an oligonucleotide having a CpG motif, an aluminum compound, or the like, the pH sensitive carrier is useful for the induction of antigen-specific CTLs.

On the other hand, in Biomaterials 2008, Vol: 29, Iss: 29, PP: 4029-4036 and Biomaterials 2013, Vol: 34, Iss: 12, PP 3042-3052, it has been reported that as a larger amount of a physiologically active substance is delivered to the cytosol, higher gene expression or more CTLs can be induced. Therefore, it is requested to achieve the delivery to the cytosol more efficiently.

Regarding a pH sensitive carrier that realizes endosome escape, it is known that when a physiologically active substance to be delivered is integrated with the carrier, the probability of being captured into the same endosome is increased, and the physiologically active substance can be delivered to the cytosol more efficiently. In WO 2013/180253 A and WO 2015/079952 A, it has been suggested that a pH sensitive carrier composed of a pH sensitive compound and an amphiphilic substance and having a membrane disruptive function promoting effect can be integrated with a physiologically active substance such as a protein or a peptide.

**SUMMARY OF INVENTION**

However, in the case of the technology described in WO 2015/079952 A, freeze-drying must be implemented in order to integrate a pH sensitive carrier and an antigen, and thus complicated processes are required. Furthermore, in the case of the technology described in WO 2013/180253 A, since the amount of the physiologically active substance that can be supported on the pH sensitive carrier is limited, it is suggested that uptake of the physiologically active substance into endosomes, the efficiency of delivery of the physiologically active substance to the cytosol, and subsequent functional expression are not sufficient. Therefore, there is a demand for a technology of integrating a physiologically active substance and a carrier more conveniently and more efficiently.

The present invention was achieved in view of the problems described above, and it is an object of the invention to provide a complex comprising a carrier and a physiologically active substance integrated together, the complex being conveniently obtainable, having excellent efficiency for the integration of the physiologically active substance, and having a satisfactory membrane disruptive function promoting effect.

The present invention intended to achieve the above-described object is a complex formed by having a physiologically active substance supported on an aggregate of a pH sensitive carrier formed by aggregating under acidic conditions.

**BRIEF DESCRIPTION OF DRAWINGS**

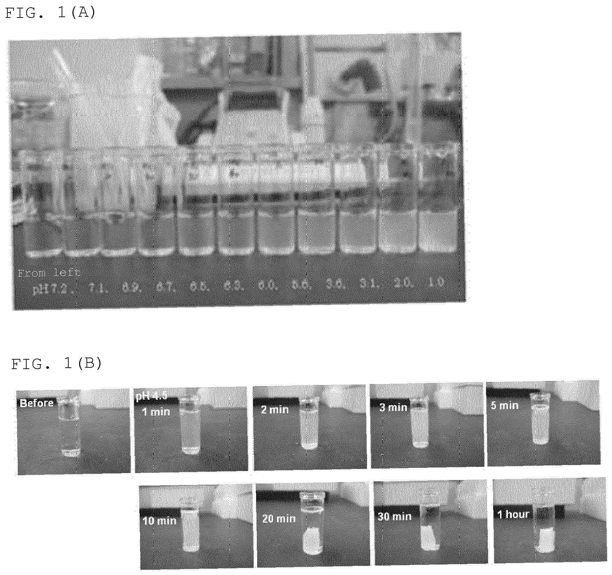

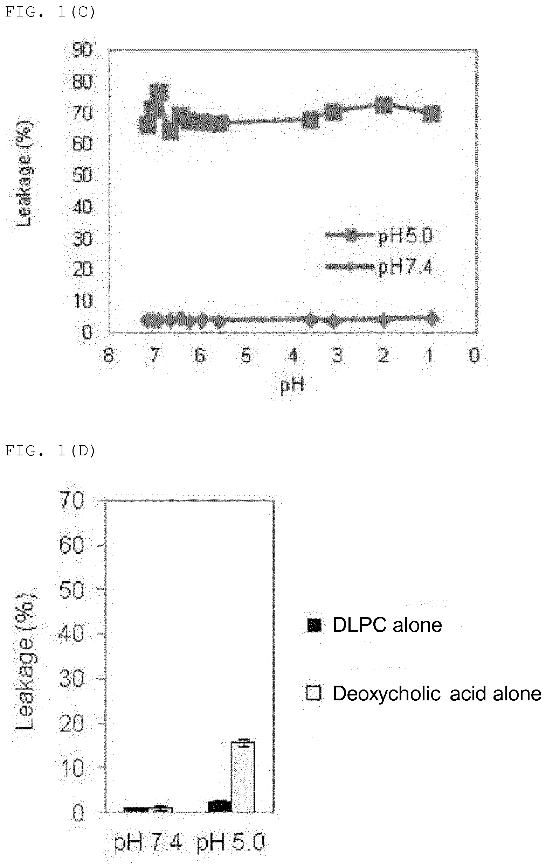

Fig. 1A is a photograph showing the external appearance obtained when a dispersion liquid of a pH sensitive carrier was set to various pH values. Fig. 1B is a photograph showing the changes over time when the pH of the dispersion liquid of a pH sensitive carrier was set to 4.5. Fig. 1C is a graph showing the leakages at pH 7.4 and pH 5.0 of aggregates of the pH sensitive carrier aggregated at various pH values. Fig. 1D is a graph showing the leakages at pH 7.4 and pH 5.0 of DLPC alone or deoxycholic acid alone.

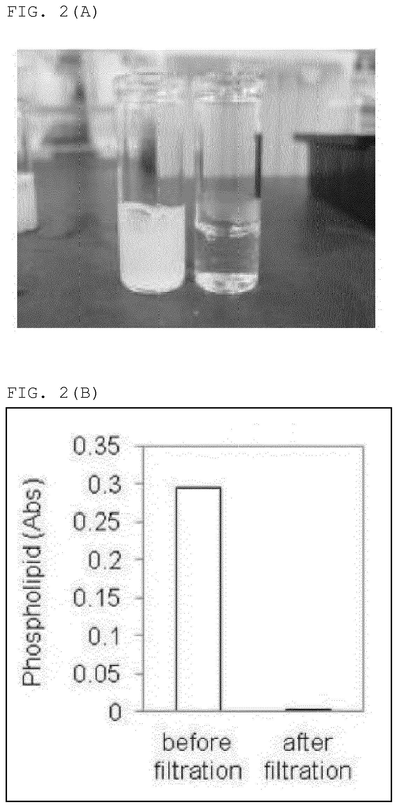

Fig. 2A is a photograph showing the external appearance before and after a filtration treatment of a mixed liquid including a pH sensitive carrier and OVA, which was acidified before the filtration treatment. Fig. 2B is a graph showing the changes in the absorbance (corresponding to the phospholipid concentration) before and after a filtration treatment of the mixed liquid including a pH sensitive carrier and OVA, which was acidified before the filtration treatment.

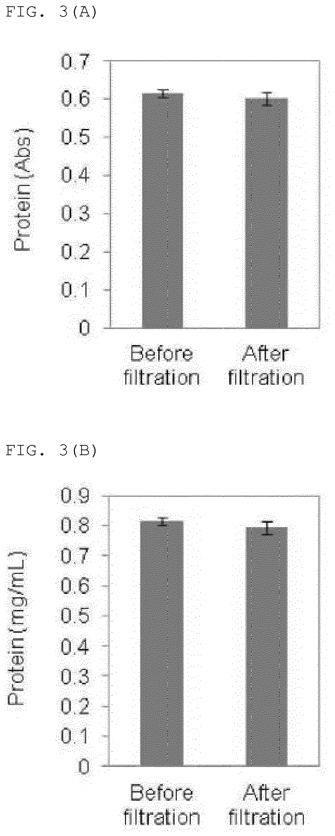

Fig. 3A is a graph showing the changes in the absorbance (corresponding to the OVA concentration) before and after a filtration treatment of a solution containing OVA. Fig. 3B is a graph presented by converting the axis of ordinate of Fig. 3A to the protein concentration.

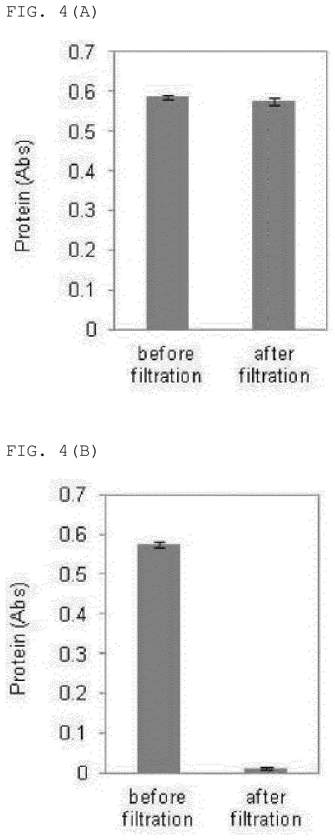

Fig. 4A is a graph showing the changes in the absorbance (corresponding to the OVA concentration) before and after a filtration treatment of a mixed liquid (pH 3.5) including OVA and a pH sensitive carrier. Fig. 4B is a graph showing the changes in the absorbance (corresponding to the OVA concentration) before and after a filtration treatment of the mixed liquid including OVA and the pH sensitive carrier, which was acidified before the filtration treatment.

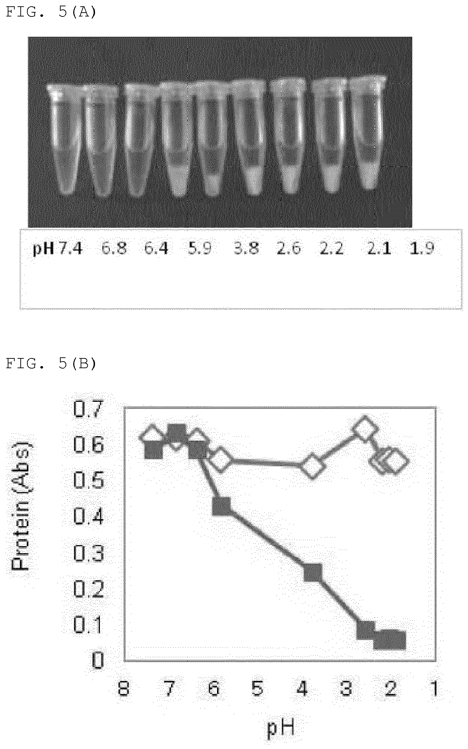

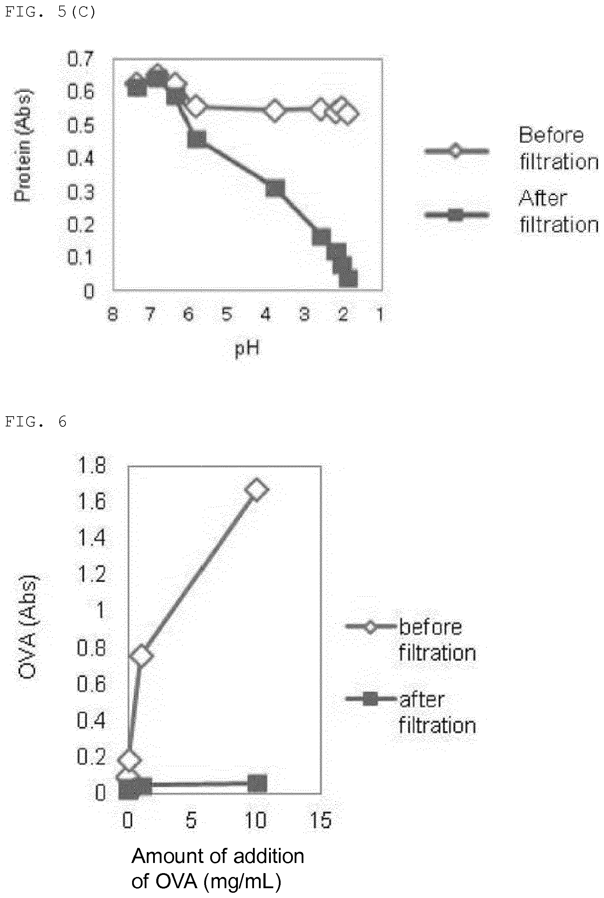

Fig. 5A is a photograph showing the external appearance obtained when the mixed liquid including OVA and a pH sensitive carrier was set to various pH values. Fig. 5B is a graph showing the changes in the absorbance (corresponding to the OVA concentration) before and after a filtration treatment of the mixed liquid including OVA and the pH sensitive carrier, which was set to various pH values and left to stand for one hour before the filtration treatment. Fig. 5C is a graph showing the changes in the absorbance (corresponding to the OVA concentration) before and after a filtration treatment of the mixed liquid including OVA and the pH sensitive carrier, which was set to various pH values and left to stand for one day before the filtration treatment.

Fig. 6 is a graph showing the changes in the absorbance (corresponding to the OVA concentration) before and after a filtration treatment of a mixed liquid including OVA at various concentrations and a pH sensitive carrier, which was acidified before the filtration treatment.

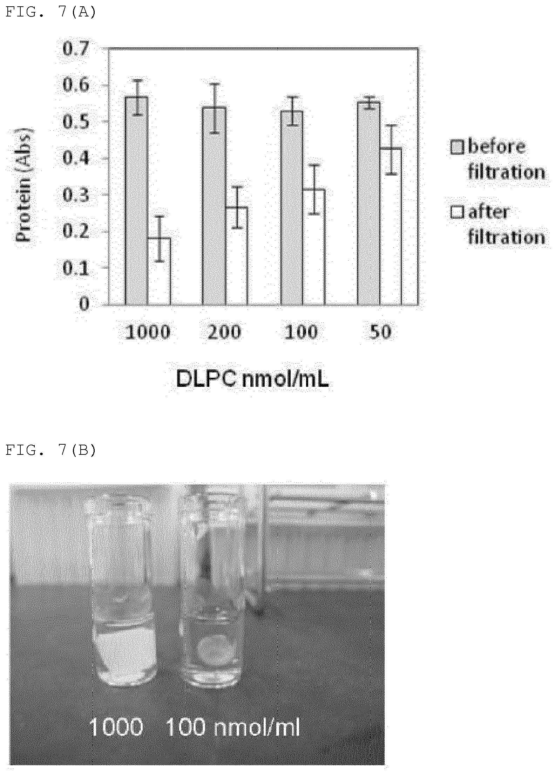

Fig. 7A is a graph showing the changes in the absorbance (corresponding to the OVA concentration) before and after a filtration treatment of mixed liquids including OVA and a pH sensitive carrier at various concentrations, which were acidified before the filtration treatment. Fig. 7B is a photograph showing the external appearance of mixed liquids including OVA and 1,000 nmol/mL or 100 nmol/mL of a pH sensitive carrier, the mixed liquid being acidified.

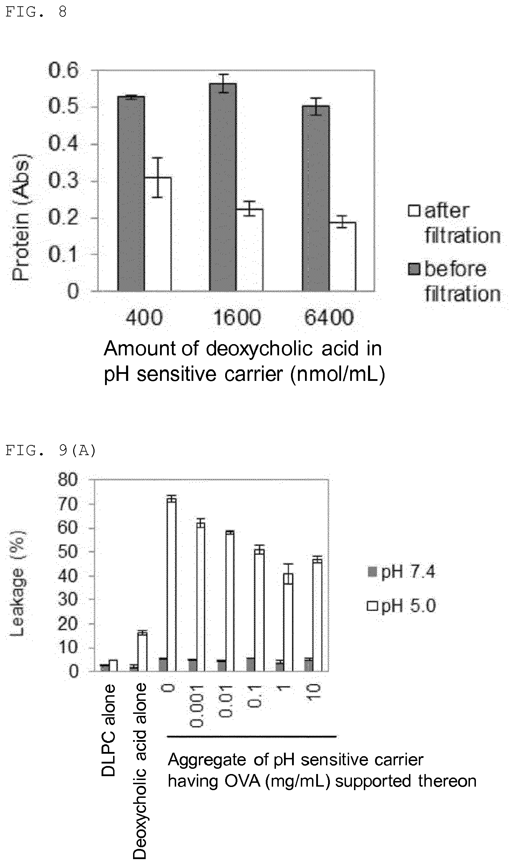

Fig. 8 is a graph showing the changes in the absorbance (corresponding to the OVA concentration) before and after a filtration treatment of a mixed liquid including a pH sensitive carrier including deoxycholic acid in various mixing amounts, and OVA.

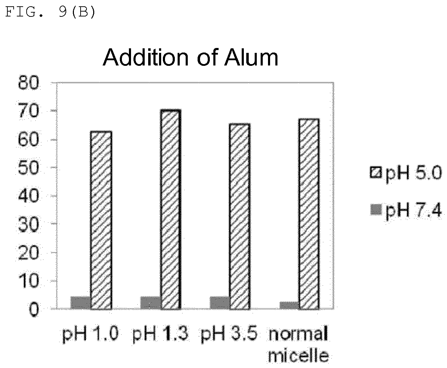

Fig. 9A is a graph showing a membrane disruptive function promoting effect of an aggregate of a pH sensitive carrier supporting OVA. Fig. 9B is a graph showing the influence on the membrane disruptive function promoting effect of a complex in the presence of aluminum hydroxide, and the axis of ordinate represents the leakage (%).

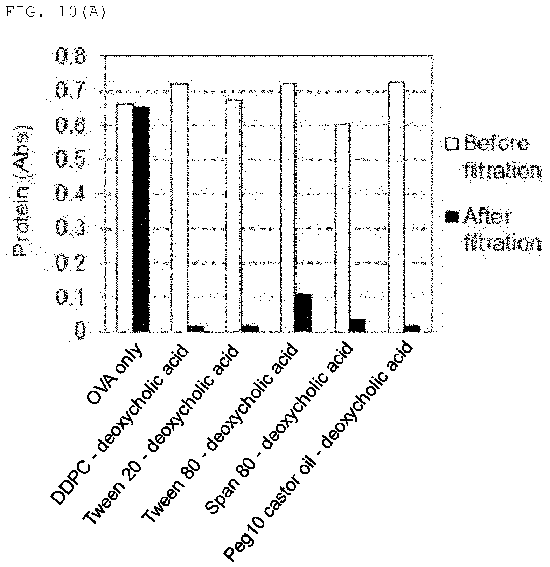

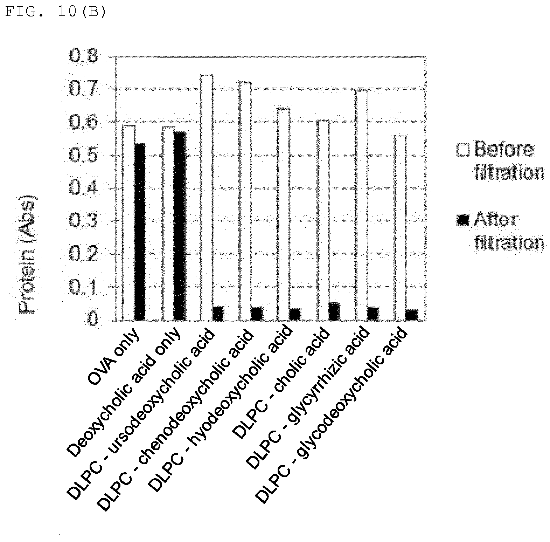

Fig. 10A is a graph showing the changes in the absorbance (corresponding to the OVA concentration) before and after a filtration treatment of solutions including pH sensitive carriers containing various amphiphilic substances, and OVA, which were acidified before the filtration treatment. Fig. 10B is a graph showing the changes in the absorbance (corresponding to the OVA concentration) before and after a filtration treatment of solutions including pH sensitive carriers containing various pH sensitive compounds, and OVA, which were acidified before the filtration treatment.

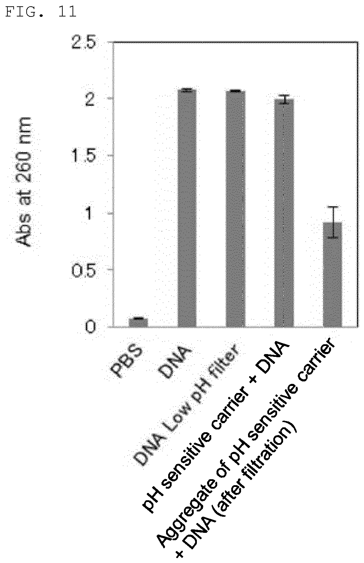

Fig. 11 is a graph showing the changes in the absorbance (corresponding to the DNA concentration) before and after a filtration treatment of a mixed liquid including a pH sensitive carrier and deoxyribonucleic acid (in the diagram, described as DNA), which was acidified before the filtration treatment.

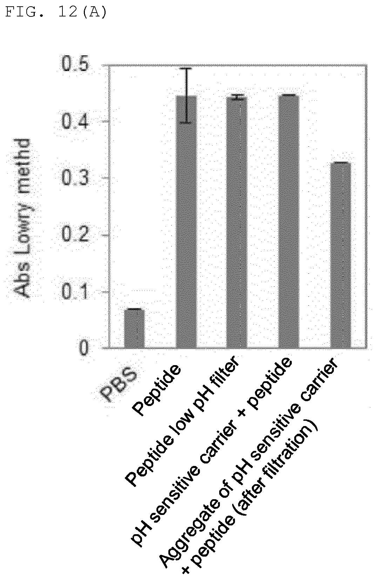

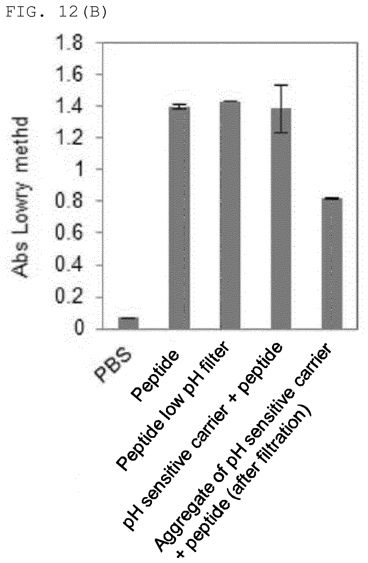

Fig. 12A is a graph showing the changes in the absorbance (corresponding to the peptide concentration) before and after a filtration treatment of a mixed liquid including a pH sensitive carrier and SIINFEKL peptide, which was acidified before the filtration treatment. Fig. 12B is a graph showing the changes in the absorbance (corresponding to the peptide concentration) before and after a filtration treatment of a mixed liquid including a pH sensitive carrier and RGPESRLL peptide, which was acidified before the filtration treatment.

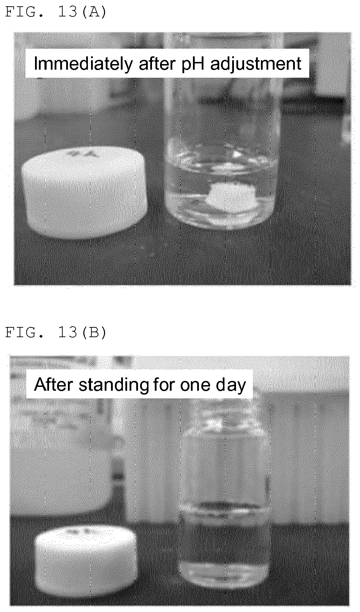

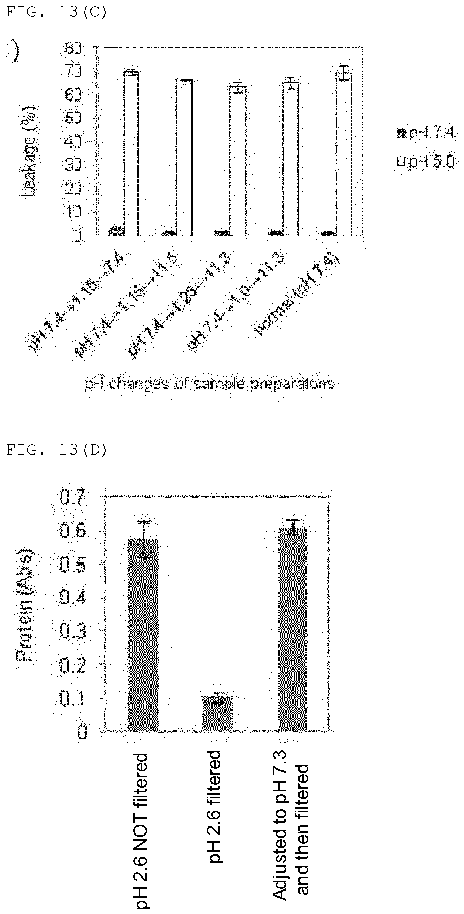

Fig. 13A is a photograph showing the external appearance of an aggregate of a pH sensitive carrier supporting OVA immediately after being left to stand at pH 7.4. Fig. 13B is a photograph showing the external appearance of the aggregate of the pH sensitive carrier supporting OVA after being left to stand for one day at pH 7.4. Fig. 13C is a graph showing the leakage obtained when the pH of a liquid including the aggregate of the pH sensitive carrier supporting OVA was changed to be neutral. Fig. 13D is a graph showing the changes in the absorbance (corresponding to the OVA concentration) before and after the changing of the pH of the liquid including the aggregate of the pH sensitive carrier supporting OVA to be neutral.

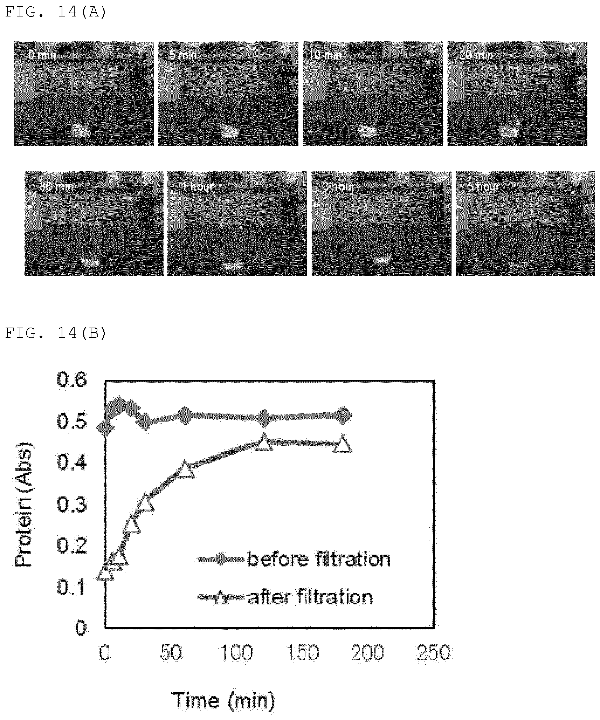

Fig. 14A is a photograph showing the changes over time in the external appearance when the pH of a liquid including an aggregate of a pH sensitive carrier supporting OVA was set to be neutral. Fig. 14B is a graph showing the changes over time in the absorbance (corresponding to the OVA concentration) when the pH of the liquid including an aggregate of a pH sensitive carrier supporting OVA was set to be neutral.

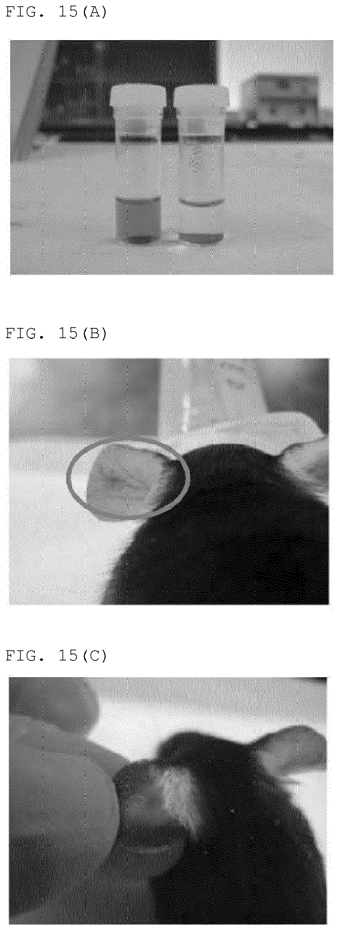

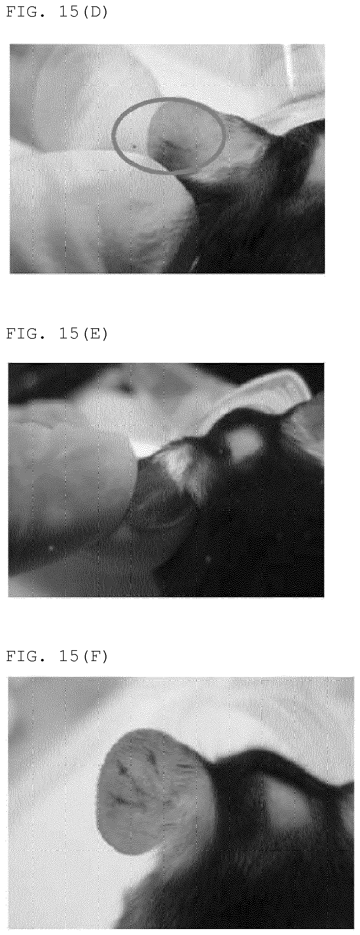

Fig. 15A is a photograph showing the external appearance of a pH sensitive carrier fluorescently modified with Rhodamine-PE and an aggregate of the pH sensitive carrier fluorescently modified with Rhodamine-PE. Fig. 15B is a photograph of a mouse to which a pH sensitive carrier fluorescently modified with Rhodamine-PE was intradermally injected. Fig. 15C is a photograph of the mouse to which the pH sensitive carrier fluorescently modified with Rhodamine-PE was intradermally injected and then exposed to excitation light. Fig. 15D is a photograph of a mouse after being intradermally injected with an aggregate of a pH sensitive carrier fluorescently modified with Rhodamine-PE. Fig. 15E is a photograph of a mouse after being intradermally injected with an aggregate of a pH sensitive carrier fluorescently modified with Rhodamine-PE and then exposed to excitation light. Fig. 15F is a photograph of a mouse after being intradermally injected several times with an aggregate of a pH sensitive carrier fluorescently modified with Rhodamine-PE.

## DESCRIPTION OF EMBODIMENTS

According to an embodiment of the present invention, a complex in which a physiologically active substance is supported on an aggregate of a pH sensitive carrier formed by aggregating under acidic conditions (hereinafter, also simply referred to as "complex") is provided. According to this embodiment, there is provided a complex in which a carrier and a physiologically active substance are integrated, the complex being conveniently obtained, having excellent efficiency for the integration of the physiologically active substance, and having a satisfactory membrane disruptive function promoting effect.

In WO 2015/079952 A, a technology of integrating a pH sensitive carrier which is composed of a pH sensitive compound and an amphiphilic substance and has a membrane disruptive function promoting effect, with an antigen as a physiologically active substance, is disclosed; however, since this technology requires freeze-drying, it cannot be said that the technology is sufficiently industrially suitable.

Furthermore, in WO 2013/180253 A, a technology of integrating a pH sensitive carrier which is composed of a pH sensitive compound and an amphiphilic substance and has a membrane disruptive function promoting effect, with a model protein of a physiologically active substance, is disclosed. However, in this technology, in the case of using a particularly large-sized physiologically active substance, the amount of the physiologically active substance that can be supported on the pH sensitive carrier is limited. Therefore, it is suggested that uptake of the physiologically active substance into endosomes, the efficiency of delivery of the physiologically active substance to the cytosol, and subsequent functional expression are not satisfactory.

Thus, the inventors of the present invention conducted an investigation on the improvement of the integration technology. As the result, the inventors found that when a pH sensitive carrier and a physiologically active substance are mixed, and the mixture is exposed to an acidic environment, a complex of the two integrated together can be conveniently obtained, and the complex has excellent efficiency for the integration of the physiologically active substance and can exhibit a satisfactory membrane disruptive function promoting effect.

The mechanism by which the above-described effect is obtained is not clearly understood; however, the mechanism is speculated as follows. Meanwhile, the present invention is not intended to be limited to the following speculation.

It is thought that the pH sensitive carrier (hereinafter, also referred to as carrier) forms an aggregate under acidic conditions, and this is thought to be mainly contributed by a hydrophobic interaction. That is, the pH sensitive carrier is formed such that a pH sensitive compound associates with a hydrophobic moiety constituted by the amphiphilic substance, and when such a pH sensitive carrier is exposed to an acidic environment, the charge of the pH sensitive compound is reduced, and consequently the associated structure becomes unstable. Thereby, structural change of the pH sensitive carrier is induced, and the pH sensitive carrier tends to be shifted to a more stable state by utilizing a hydrophobic interaction as the driving force. As a result, it is considered that an aggregate participated by a large number of pH sensitive carriers are formed. Therefore, it is thought that when a hydrophobic physiologically active substance is mixed with this carrier, and the mixture is exposed to an acidic environment, the physiologically active substance is co-aggregated with the carrier through hydrophobic interaction and is incorporated into the aggregate formed by the carrier. Furthermore, as described above, since the associated form of the pH sensitive carrier is unstable under acidic conditions, it is thought that when the pH sensitive carrier contacts with another carrier, rearrangement of the amphiphilic substance occurs between the mutual carriers, and the carriers can easily fuse with each other. Thereby, it is speculated that the pH sensitive carrier is aggregated while forming a crosslinked structure with other carriers. Therefore, when a carrier and a physiologically active substance are mixed, and the mixture is exposed to an acidic environment, the physiologically active substance is physically captured by a crosslinked structure of the aggregate of the carrier, regardless of the hydrophilicity / hydrophobicity of the physiologically active substance. At this time, a large-sized physiologically active substance such as a protein cannot easily escape from the crosslinked structure of the aggregate of the carrier, and therefore, the physiologically active substance is easily captured within the aggregate.

By doing as described above, it is thought that when a pH sensitive carrier and a physiologically active substance are mixed, and the mixture is exposed to an acidic environment, a complex in which the two are integrated can be conveniently obtained. Furthermore, it is thought that this complex has excellent efficiency for the integration with physiologically active substances (particularly, hydrophobic physiologically active substances and large-sized physiologically active substances).

Furthermore, it is speculated that the pH sensitive carrier retains an associated form between a pH sensitive compound and an amphiphilic substance to a certain extent even if the pH sensitive carrier is aggregated. Therefore, in a system in which the complex of the present invention and a biological membrane (for example, a cell membrane or a vesicle membrane) are present, when the pH becomes less than the physiological pH, the associated form of the aggregate of the pH sensitive carrier constituting the complex is changed, and after coming into contact with the biological membrane, the biological membrane also undergoes a change in the membrane structure as a result of the change in the associated form. Therefore, the complex of the present invention can exhibit a satisfactory membrane disruptive function promoting effect.

Hereinafter, embodiments of the present invention will be described. Meanwhile, the present invention is not intended to be limited to the following embodiments only.

According to the present specification, the "aggregate" is not particularly limited as long as it has a structure formed by a plurality of pH sensitive carriers gather together, and the aggregate may be irregularly shape or regularly shaped. At this time, the structure of the individual pH sensitive carriers may be retained, or a plurality of carriers may be fused. The form of the aggregate can be checked by, for example, known observation means such as a transmission electron microscope (TEM) and a scanning probe microscope (SPM). In the present specification, when a solution containing a pH sensitive carrier is treated with a filter having a pore size of 0.22 $\mu$m, and the phospholipid concentration of the liquid that has passed through the filter is less than the phospholipid concentration of the liquid before being passed through the filter, it is considered that an aggregate of the pH sensitive carrier has been formed. This phospholipid concentration can be determined using a known phospholipid quantification means such as TEST WAKO. In a case in which quantitative determination of the phospholipid is performed using TEST WAKO, the procedure will follow the protocol of the manufacturer.

According to the present specification, the term "support" means that a physiologically active substance is retained inside an aggregate formed by a pH sensitive carrier aggregating under acidic conditions, by means of physical capture (for example, captured in a crosslinked structure) or chemical bonding (for example, hydrophobic interaction, ionic bonding, or hydrogen bonding). At this time, the state of presence of the physiologically active substance within the aggregate is not particularly limited, and the physiologically active substance may be present between a membrane of the pH sensitive carrier forming the aggregate and a membrane of another carrier forming the aggregate, or may be inserted into a membrane of a carrier.

Herein, the term "membrane disruptive function" means a function of causing leakage in a leaching test. Herein, the leaching test in the present specification is a test in which liposomes (dispersion) including an aqueous solution containing a quenching substance and a fluorescent substance, and an evaluation sample dispersion such as a solution containing a complex (aggregate of a pH sensitive carrier supporting a physiologically active substance) are added to an aqueous solution whose pH is adjusted to a predetermined level, followed by incubating the aqueous solution at 37°C for 90 minutes or 30 minutes and measuring the fluorescence of the aqueous solution. According to this method, an amount of a fluorescent substance leached out from the liposomes can be measured, from which the liposome membrane disruptive function of the complex can be confirmed. By having such a membrane disruptive function, a component that can be incorporated inside the membrane of a biological membrane (for example, endosome) can be leached to the outside of the biological membrane (for example, cytoplasmic matrix). Incidentally, the leaching test will be described in more detail in Examples described later.

Herein, the expression "to develop a membrane disruptive function promoting effect" means to satisfy both of requirements (1) and (2): (1) in the leaching test, a leakage at a predetermined pH that is less than a physiological pH increases compared to a leakage at the physiological pH and the amount of increase is larger than the amount of increase in a case where the pH sensitive compound alone is subjected to the test; and (2) in the leaching test at a predetermined pH less than the physiological pH, a leakage of the complex is more than the sum of a leakage of the pH sensitive compound alone and a leakage of the amphipathic substance alone. More specifically, to develop a membrane disruptive function promoting effect means that, in the leaching test at a pH of 7.4 and at a pH of 5.0 or 4.5, a leakage Lc of the complex, a leakage La of the pH sensitive compound alone, and a leakage Lb of the amphipathic substance alone satisfy both the following relations. That is, the above (1) is represented by the following Formula (1) and the above (2) is represented by the following Formula (2). Incidentally, in the following formulas, leakages at a pH of 7.4 are, respectively, denoted by $Lc_{7.4}$, $La_{7.4}$, and $Lb_{7.4}$, and leakages at a pH of 5.0 or 4.5 are, respectively, denoted by $Lc_x$, $La_x$, and $Lb_x$.
[Math. 1]

$$\text{Formula (1)} \quad \Delta = (Lc_x - Lc_{7.4}) - (La_x - La_{7.4}) > 0$$

$$\text{Formula (2)} \quad \Delta' = Lc_x - (La_x + Lb_x) > 0$$

In formula (1) described above, $\Delta$ may exceed 0; however, $\Delta$ is preferably 5 or more, more preferably 10 or more, and even more preferably 30 or more. Furthermore, in formula (2) described above, $\Delta'$ may exceed 0; however, $\Delta'$ is preferably 5 or more, more preferably 10 or more, and even more preferably 15 or more.

Herein, the phrase "under acidic conditions" means less than pH 7.0, and preferably less than pH 6.4.

Herein, the term "physiological pH" means a pH in a normal tissue or normal body fluid. The physiological pH is generally 7.4 and differs slightly ($\pm 0.1$) depending on the normal tissue or normal body fluid. In addition, the term "a predetermined pH less than a physiological pH" may be a pH of less than 7.4, and is preferably a pH of 3.0 or more and a pH of less than 7.4, more preferably a pH of 4.0 or more and a pH of less than 7.3, and further preferably a pH of 4.5 or more and a pH of less than 7.0.

Herein, the term "neutral pH" means pH 6 or more and pH 8 or less (specifically, pH 6.0 or more and pH 8.0 or less).

Herein, "X to Y" indicating the range means "X or more and Y or less" including X and Y. In addition, in the present specification, unless otherwise stated, the operations and the measurements of physical properties are conducted under the conditions of room temperature (from 20°C to 25°C)/relative humidity of from 40% RH to 50% RH.

<Constituent components of complex>

[pH sensitive carrier]

The complex of the present invention comprises a physiologically active substance supported on an aggregate of a pH sensitive carrier formed by aggregating under acidic conditions, and the pH sensitive carrier is composed mainly of a pH sensitive compound and an amphiphilic substance.

(pH sensitive compound)

The pH sensitive compound is at least one selected from the group consisting of deoxycholic acid, cholic acid, ursodeoxycholic acid, chenodeoxycholic acid, hyodeoxycholic acid, higher bile acids, glycodeoxycholic acid, glycyrrhizic acid, glycyrrhetinic acid, and salts thereof. Among them, the pH sensitive compound is more preferably at least one selected from the group consisting of deoxycholic acid, cholic acid, ursodeoxycholic acid, chenodeoxycholic acid, hyodeoxycholic acid, glycodeoxycholic acid, glycyrrhizic acid, and salts thereof. Examples of a salt of the pH sensitive compound include, but are not particularly limited to, alkali metal salts of lithium, sodium, potassium, and the like; alkaline earth metal salts of magnesium, calcium, barium, and the like; ammonium salts; and the like. These pH sensitive compounds may be used singly, or two or more kinds thereof may be used in combination.

The deoxycholic acid, cholic acid, ursodeoxycholic acid, chenodeoxycholic acid, hyodeoxycholic acid, higher bile acid, and glycodeoxycholic acid, which are preferably used in the present invention, are generally called "bile acid." The bile acid has been known for long as a typical steroid derivative and has been utilized in the field of bacteriology. The bile acid forms complexes with cholesterol, lipids and fat-soluble vitamins in the human body and has a role of supplementing absorption thereof. Moreover, because of the capability of forming complexes with lipids, proteins, and hydrophobic materials in view of physicochemical properties of the bile acid, it has been long utilized for isolation and purification of proteins and also as a solubilizer or emulsifier. In recent years, attention has been paid to the use in a preparation process of vaccine and also to as an absorption enhancer for drug through a bile acid transporter. In particular, sodium deoxycholate (also known as sodium desoxycholate) and ursodeoxycholic acid (also known as ursodesoxycholic acid) have been approved as a pharmaceutical additive capable of being injected to humans, respectively, and their superior safety performance has been recognized. Therefore, as the pH sensitive compound, deoxycholic acid, ursodeoxycholic acid, or salts thereof (for example, sodium salts) are further preferably used. Among them, from the viewpoint of having excellent pH-responsiveness, deoxycholic acid is particularly preferred.

It is preferable that the pH sensitive compound is contained at a proportion of 10 mol or more with respect to 100 mol of the amphiphilic substance. The proportion is more preferably 10 to 640 mol, further preferably 20 to 320 mol, and particularly preferably 20 to 160 mol, with respect to 100 mol of the amphiphilic substance.

(Amphiphilic substance)

The amphipathic substance is preferably at least one selected from the group consisting of a phosphatidylcholine having 10 to 12 carbon atoms, a polyoxyethylene sorbitan monofatty acid ester having 12 to 18 carbon atoms, a sorbitan fatty acid ester having 16 to 18 carbon atoms, glycerol monooleate, glycerol dilaurate, glycerol distearate, glycerol dioleate, polyoxyethylene castor oil, and $\alpha$-tocopherol. These amphipathic substances may be used singly or in combination of two or more kinds thereof. Incidentally, in the present specification, the "number of carbon atoms" in the amphipathic substance means the number of carbon atoms in the fatty acid component (acyl group) constituting the hydrophobic moiety of the amphipathic substance.

As the phosphatidylcholine having 10 to 12 carbon atoms, diacylphosphatidylcholine having a saturated acyl group is preferred, and examples thereof include didecanoylphosphatidylcholine (DDPC; 1,2-didecanoyl-sn-glycero-3-phosphatidylcholine), and dilauroylphosphatidylcholine (DLPC; 1,2-dilauroyl-sn-glycero-3-phosphatidylcholine). As the phosphatidylcholine, a naturally-derived one or a synthesized one obtained by a known method may be used, or commercially available ones can be used.

Examples of the polyoxyethylene sorbitan monofatty acid ester having 12 to 18 carbon atoms include polyoxyethylene sorbitan monolauric acid ester (polyoxyethylene sorbitan monolaurate), polyoxyethylene sorbitan myristic acid ester (polyoxyethylene sorbitan monomyristate), polyoxyethylene sorbitan monopalmitic acid ester (polyoxyethylene sorbitan palmitate), polyoxyethylene sorbitan monostearic acid ester (polyoxyethylene sorbitan monostearate), polyoxyethylene sorbitan monooleic acid ester (polyoxyethylene sorbitan monooleate), and the like. Although the degree of polymerization of polyoxyethylene is not particularly limited, the degree of polymerization with respect to the total of polyoxyethylene chains added to sorbitan is preferably 10 to 200, more preferably 15 to 100, and further preferably 20 to 50. As the polyoxyethylene sorbitan monofatty acid ester, a synthetized one may be used or a commercial one may be used. As a commercial product of the polyoxyethylene sorbitan monofatty acid ester, there can be preferably used, for example, those commercially sold under the designations of Tween 20 (polyoxyethylene sorbitan monolauric acid ester), Tween 40 (polyoxyethylene sorbitan monopalmitic acid ester), Tween 60 (polyoxyethylene sorbitan monostearic acid ester), and Tween 80 (polyoxyethylene sorbitan monooleic acid ester).

Examples of the sorbitan fatty acid ester having 16 to 18 carbon atoms include sorbitan monofatty acid esters such as sorbitan monopalmitic acid ester (sorbitan monopalmitate), sorbitan monostearic acid ester (sorbitan monostearate), and sorbitan monooleic acid ester (sorbitan monooleate); sorbitan trifatty acid esters such as sorbitan tripalmitic acid ester (sorbitan tripalmitate), sorbitan tristearic acid ester (sorbitan tristearate), and sorbitan trioleic acid ester (sorbitan trioleate); and the like. As the sorbitan fatty acid ester, a synthetized one may be used or a commercial one may be used. As a commercial product of the sorbitan fatty acid ester, there can be preferably used, for example, those commercially sold under the designations of SPAN 40 (sorbitan palmitic acid ester), SPAN 60 (sorbitan stearic acid ester), SPAN 80 (sorbitan oleic acid ester), SPAN 65 (sorbitan tristearic acid ester), and SPAN 85 (sorbitan trioleic acid ester). Among these, SPAN 80, SPAN 65, and SPAN 85 are preferably used.

The glycerol monooleate (glyceryl monooleate), glycerol dilaurate (glyceryl dilaurate), glycerol distearate (glyceryl distearate), and glycerol dioleate (glyceryl dioleate) are acyl glycerols in which one or two molecules of a fatty acid are ester-bound to glycerin, and the sites at which the fatty acid is bound are not particularly limited. For example, with glycerol monooleate that is a monoacyl glycerol, the fatty acid may be ester-bound to at the C1 or C2 position of glycerin. Further, with glycerol dilaurate, glycerol distearate, and glycerol dioleate that are each a diacyl glycerol, the fatty acid may be ester-bound to at the C1 and C2 positions or at the C1 and C3 positions of glycerin. For example, as glycerol dilaurate, $\alpha,\alpha'$-dilaurin which is substituted at the C1 and C3 positions is preferred. As glycerol distearate or glycerol dioleate, diacyl glycerol which is substituted at the C1 and C2 positions is preferred. As these glycerol derivatives, a synthetized one may be used or a commercial one may be used.

As a polyoxyethylene castor oil, mention is made of adducts of polyoxyethylenes to castor oil. The degree of polymerization of polyoxyethylene is not particularly limited, but is preferably 3 to 200, more preferably 5 to 100, and further preferably 10 to 50. As the polyoxyethylene castor oil, a synthetized one may be used or a commercial one may be used.

As $\alpha$-tocopherol, a naturally-derived one or a synthesized one obtained by a known method may be used, or commercially available ones may be used.

According to an embodiment of the present invention, the amphiphilic substance is preferably phosphatidylcholine having 10 to 12 carbon atoms, and particularly preferably dilauroylphosphatidylcholine (DLPC) having 12 carbon atoms.

(Combination of pH sensitive compound and amphiphilic substance)

The pH sensitive carrier can develop a membrane disruptive function promoting effect at a desired pH by the combination of a pH sensitive compound and an amphipathic substance. At this time, the pH at which the pH sensitive carrier commences to develop the membrane disruptive function promoting effect differs depending on the combination of a pH sensitive compound and an amphipathic substance. This is considered for the following reasons: pKa differs depending on the type of pH sensitive compound and the manner of forming association with an amphipathic substance also differs depending on the combination of a pH sensitive compound and an amphipathic substance. Therefore, when a combination of a pH sensitive compound and an amphipathic substance is appropriately changed, the proper choice of the pH at which the function can be developed is possible, and it is possible to set up the delivery in vivo and the intracellular delivery in detail.

With regard to the pH sensitive carrier of the present invention, preferred examples of the combination of the pH sensitive compound and the amphiphilic substance include deoxycholic acid and DDPC, deoxycholic acid and DLPC, deoxycholic acid and Tween 20, deoxycholic acid and Tween 40, deoxycholic acid and Tween 60, deoxycholic acid Tween 80, deoxycholic acid and SPAN 40, deoxycholic acid and SPAN 60, deoxycholic acid and SPAN 80, deoxycholic acid and SPAN 65, deoxycholic acid and SPAN 85, deoxycholic acid and $\alpha$-tocopherol, deoxycholic acid and glycerol monooleate, deoxycholic acid and glycerol distearate, deoxycholic acid and glycerol dioleate, deoxycholic acid and glycerol dilaurate ($\alpha,\alpha$'-dilaurin), deoxycholic acid and polyoxyethylene castor oil (preferably, PEG10 castor oil), ursodeoxycholic acid and DDPC, ursodeoxycholic acid and DLPC, ursodeoxycholic acid and Tween 20, ursodeoxycholic acid and Tween 40, ursodeoxycholic acid and Tween 60, ursodeoxycholic acid and Tween 80, ursodeoxycholic acid and SPAN 40, ursodeoxycholic acid and SPAN 60, ursodeoxycholic acid and SPAN 80, ursodeoxycholic acid and SPAN 65, ursodeoxycholic acid and SPAN 85, ursodeoxycholic acid and $\alpha$-tocopherol, ursodeoxycholic acid and glycerol monooleate, ursodeoxycholic acid and glycerol distearate, ursodeoxycholic acid and glycerol dioleate, ursodeoxycholic acid and glycerol dilaurate ($\alpha,\alpha$'-dilaurin), ursodeoxycholic acid and polyoxyethylene castor oil, chenodeoxycholic acid and DLPC, hyodeoxycholic acid and DLPC, cholic acid and DLPC, glycyrrhizic acid and DDPC, glycyrrhizic acid and DLPC, glycyrrhizic acid and Tween 20, glycyrrhizic acid and Tween 40, glycyrrhizic acid and Tween 60, glycyrrhizic acid and Tween 80, glycyrrhizic acid and SPAN 40, glycyrrhizic acid and SPAN 60, glycyrrhizic acid SPAN 80, glycyrrhizic acid and SPAN 65, glycyrrhizic acid and SPAN 85, glycyrrhizic acid and $\alpha$-tocopherol, glycyrrhizic acid and glycerol monooleate, glycyrrhizic acid and glycerol distearate, glycyrrhizic acid and glycerol dioleate, glycyrrhizic acid and glycerol dilaurate ($\alpha,\alpha$'-dilaurin), glycyrrhizic acid and polyoxyethylene castor oil, and glycodeoxycholic acid and DLPC are preferred. In the above description, the pH sensitive compound may be a salt.

More preferred are deoxycholic acid and DDPC, deoxycholic acid and DLPC, deoxycholic acid and Tween 20, deoxycholic acid and Tween 40, deoxycholic acid and Tween 60, deoxycholic acid and Tween 80, deoxycholic acid and SPAN 40, deoxycholic acid and SPAN 65, deoxycholic acid and SPAN 80, deoxycholic acid and SPAN 85, deoxycholic acid and PEG10 castor oil, deoxycholic acid and $\alpha$-tocopherol, deoxycholic acid and monoolein, deoxycholic acid and polyoxyethylene castor oil, ursodeoxycholic acid and DDPC, ursodeoxycholic acid and DLPC, ursodeoxycholic acid and Tween 40, ursodeoxycholic acid and Tween 60, ursodeoxycholic acid and Tween 80, ursodeoxycholic acid and SPAN 40, ursodeoxycholic acid and SPAN 65, ursodeoxycholic acid and SPAN 85, ursodeoxycholic acid and $\alpha$-tocopherol, ursodeoxycholic acid and monoolein, ursodeoxycholic acid and polyoxyethylene castor oil, chenodeoxycholic acid and DLPC, hyodeoxycholic acid and DLPC, cholic acid and DLPC, glycyrrhizic acid and DDPC, glycyrrhizic acid and DLPC, glycyrrhizic acid and Tween 40, glycyrrhizic acid and Tween 60, glycyrrhizic acid and Tween 80, glycyrrhizic acid and SPAN 40, glycyrrhizic acid and SPAN 65, glycyrrhizic acid and SPAN 85, glycyrrhizic acid and $\alpha$-tocopherol, glycyrrhizic acid and monoolein, glycyrrhizic acid and polyoxyethylene castor oil, and glycodeoxycholic acid and DLPC. In the above description, the pH sensitive compound may be a salt.

Among them, combinations of deoxycholic acid and DDPC, deoxycholic acid and DLPC, deoxycholic acid and Tween 20, deoxycholic acid and Tween 80, deoxycholic acid and SPAN 80, deoxycholic acid and PEG10 castor oil, ursodeoxycholic acid and DLPC, chenodeoxycholic acid and DLPC, hyodeoxycholic acid and DLPC, cholic acid and DLPC, glycyrrhizic acid and DLPC, and glycodeoxycholic acid and DLPC are preferred. In the above description, the pH sensitive compound may be a salt.

That is, according to a preferred embodiment of the present invention, the pH sensitive carrier includes at least one pH sensitive compound selected from the group consisting of deoxycholic acid, cholic acid, ursodeoxycholic acid, chenodeoxycholic acid, hyodeoxycholic acid, higher bile acids, glycodeoxycholic acid, glycyrrhizic acid, glycyrrhetinic acid, and salts thereof; and at least one amphiphilic substance selected from the group consisting of phosphatidylcholine having 10 to 12 carbon atoms, a polyoxyethylene sorbitan monofatty acid ester having 12 to 18 carbon atoms, a sorbitan fatty acid ester having 16 to 18 carbon atoms, glycerol monooleate, glycerol dilaurate, glycerol distearate, glycerol dioleate, polyoxyethylene castor oil, and $\alpha$-tocopherol. In the above description, the pH sensitive compound may be a salt.

[Physiologically active substance]

The complex of the present invention has excellent efficiency for integration of the physiologically active substance and exhibits a membrane disruptive function promoting effect at a site where the pH has decreased. Therefore, the complex can selectively deliver a physiologically active substance to a site to be treated, such as an inflammation site. Furthermore, the complex of the present invention can deliver a physiologically active substance to the cytosol of cells.

The type of the physiologically active substance to be included in the complex is not particularly limited, and examples include a nucleic acid, a low molecular weight compound, a protein, and a peptide. Among them, from the viewpoint that excellent efficacy is exhibited within cells, the physiologically active substance is preferably a protein, a peptide, or a nucleic acid.

Examples of the nucleic acid include nucleic acids having therapeutic effects, such as siRNA, ODN (oligodeoxynucleotide), and deoxyribonucleic acid (DNA). The length of the nucleic acid is preferably 500 bp or more.

Examples of the low molecular weight compound include cytotoxic agents such as mitomycin, docetaxel, and methotrexate; prodrugs such as 5-aminolevulinic acid and protoporphyrin IX; anti-inflammatory agents such as ganciclovir, dexamethasone, ribavirin, and vidarabine; contrast agents such as DOTA (1,4,7,10-tetraazacyclotetradecane-N,N',N'',N'''-tetraacetic acid) and DTPA (1,4,7,10-tetraazacyclodecane-N,N',N'',N'''-tetraacetic acid); and neuroprotective agents such as edaravone.

Examples of the protein include oxidation-reduction enzymes such as SOD (Superoxide dismutase) and indophenol oxidase; cytokines such as IL-10; growth factors such as b-FGF; thrombolytic agents such as t-PA; hormones such as erythropoietin; cell death inhibitory proteins such as PSD (Postsynaptic density protein) and FNK (Anti cell death Factor); and antibodies such as Fab (Fragment), IgG, and IgE. The molecular weight of the protein is preferably 5 to 200 kDa.

Examples of the peptide include peptide drugs such as cyclosporine A and JIP-1 (JNK-interracting protein 1). Examples of the peptide also include tumor antigen peptides. The molecular weight of the peptide is preferably 5,000 to 45,000, and more preferably 6,000 to 20,000.

The size of the physiologically active substance is not particularly limited; however, as the size is larger, it is preferable because the physiologically active substance can be easily captured by a crosslinked structure formed by an aggregate of the pH sensitive carrier.

The physiologically active substance may be a hydrophilic substance, or may be a hydrophobic substance. Among these, when the physiologically active substance is a hydrophobic substance, the physiologically active substance can be easily incorporated into the inside of the aggregate of the pH sensitive carrier by hydrophobic interaction. Therefore, a hydrophobic substance is preferable.

A suitable supporting amount of the physiologically active substance in the complex of the present invention is, for example, 0.001 to 10 mg with respect to 1,000 nmol of the amphiphilic substance constituting the pH sensitive carrier. Meanwhile, a more suitable supporting amount can be selected as appropriate according to the type of the physiologically active substance.

<Method for producing complex>

The complex according to the present invention can be obtained by mixing the above-described pH sensitive carrier and physiologically active substance, and exposing this mixture to an acidic environment. Therefore, for example, the complex of the present invention can be obtained in a liquid medium by preparing a mixed liquid including the pH sensitive carrier and a physiologically active substance and acidifying the pH of the mixed liquid. Therefore, the present invention also provides a method for producing a complex, the method including preparing a pH sensitive carrier containing a pH sensitive compound and an amphiphilic substance (step 1), mixing the pH sensitive carrier and a physiologically active substance to prepare a mixed liquid (step 2), and adjusting the pH of the mixed liquid to a value of less than 6.4 (step 3).

Various steps of the method for producing the complex according to the present invention will be described below.

[Step 1: step of preparing pH sensitive carrier]

In this step, a pH sensitive carrier is prepared by associating the above-described pH sensitive compound with the above-described amphiphilic substance.

Regarding the method of associating the pH sensitive compound with the amphiphilic substance, the pH sensitive compound and the amphiphilic substance may be brought into contact in an aqueous solution. Specifically, an aqueous solution containing a pH sensitive compound and an amphiphilic substance is prepared, and this solution is dispersed by strongly stirring the solution using an emulsifying machine, a vortex mixer, ultrasonic waves, or the like. Thereby, a dispersion liquid of a pH sensitive carrier in which the pH sensitive compound is associated with the amphiphilic substance can be obtained.

More specifically, (1) a method including preparing an aqueous solution containing a pH sensitive compound and an aqueous solution containing an amphiphilic substance separately, mixing these aqueous solutions, dispersing this solution by strongly stirring the solution using an emulsifying machine, a vortex mixer, ultrasonic waves, or the like, and thereby obtaining a pH sensitive carrier; and (2) a method of preparing a pH sensitive carrier by the Bangham method, which is known as a production method for liposomes; may be mentioned. Regarding the Bangham method, specifically, constituent components of the pH sensitive carrier, such as a pH sensitive compound and an amphiphilic substance, are dissolved in an organic solvent (for example, methanol or chloroform) in a glass vessel, the organic solvent is removed by means of a rotary evaporator or the like, and thereby a thin film is formed on the wall of the glass vessel. Next, the aqueous solution is introduced into the glass vessel in which a thin film has been formed, the thin film is allowed to swell at normal temperature (5°C to 35°C), and the glass vessel is agitated at normal temperature (5°C to 35°C) . At this time, the aqueous solution is strongly stirred using an emulsifying machine, a vortex mixer, or ultrasonic waves, and thereby the thin film can be sufficiently dispersed in the aqueous solution. Furthermore, in the production method of (1) described above, the pH sensitive compound may be mixed into an aqueous solution containing an amphiphilic substance. Meanwhile, regarding the solvent of the aqueous solution, the solvent of the aqueous solution mentioned above can be used.

Regarding the details of the Bangham method, a known production method for liposomes can be referred to, and the method is described in "Liposomes" (NOJIMA Shoshichi, SUNAMOTO Junzo, and INOUE Keizo, ed. Nankodo Co., Ltd.) and "Liposomes in Life Science" (TERADA Hiroshi, and YOSHIMURA Tetsuro, ed., Springer-Verlag Tokyo, Inc.).

It is preferable that the pH sensitive compound is mixed at a proportion of 10 mol or more with respect to 100 mol of the amphiphilic substance. Above all, from the viewpoint of further enhancement of the efficiency for integration of the physiologically active substance, the mixing amount of the pH sensitive compound is preferably from 40 mol to 640 mol, more preferably from 160 mol to 640 mol, and even more preferably 320 mol to 640 mol, with respect to 100 mol of the amphiphilic substance.

The concentration of the amphiphilic substance in the aqueous solution is preferably set to 6 to 2,000 nmol/mL, and more preferably to 10 to 1,000 nmol/mL.

The concentration of the pH sensitive compound in the aqueous solution is preferably set to 400 to 6,400 nmol/mL.

(Solvent)

Regarding the solvent used for the above-described aqueous solution, an aqueous solution containing a buffer, NaCl, and sugars such as glucose and sucrose, is preferred.

As the buffer, any known buffer can be appropriately used as long as the buffer can maintain the pH of the aqueous solution containing the pH sensitive carrier to be higher than or equal to a physiological pH, and there are no particular limitations. Examples of the buffer that can be used include a phosphoric acid buffer, a citric acid buffer, a citric acid-phosphoric acid buffer, GOOD buffers such as a trishydroxymethylaminomethane-HCl buffer (Tris-hydrochloric acid buffer), a MES buffer (2-morpholinoethanesulfonic acid buffer), a TES buffer (N-tris(hydroxymethyl)methyl-2-aminoethanesulfonic acid buffer), an acetic acid buffer, a MOPS buffer (3-morpholinopropanesulfonic acid buffer), a MOPS-NaOH buffer, a HEPES buffer (4-(2-hydroxyethyl)-1-piperazineethanesulfonic acid buffer), and a HEPES-NaOH buffer; amino acid-based buffers such as a glycine-hydrochloric acid buffer, a glycine-NaOH buffer, a glycylglycine-NaOH buffer, and a glycylglycine-KOH buffer; boric acid-based buffers such as a Tris-boric acid buffer, a boric acid-NaOH buffer, and a boric acid buffer; an imidazole buffer; and the like. Among these, a phosphoric acid buffer, a citric acid buffer, a citric acid-phosphoric acid buffer, a Tris-hydrochloric acid buffer, a MES buffer, an acetic acid buffer, and a HEPES-NaOH buffer are preferred. The concentration of the buffer is not particularly limited, and the concentration is preferably 0.1 to 200 mM, and more preferably 1 to 100 mM. Meanwhile, the concentration of the buffer in the present invention means the concentration (mM) of the buffer include in the aqueous solution.

The concentration of NaCl or sugars such as glucose and sucrose is not particularly limited, and the concentration is preferably 0.1 to 200 mM, and more preferably 1 to 150 mM.

In this step, additives such as a stabilizer may be added to the aqueous solution. The content of the additives is not particularly limited as long as the additives do not destroy the pH sensitive carrier; however, the content is preferably 150 mol or less, and more preferably 66.4 mol or less, with respect to 100 mol of the amphiphilic substance.

(Stabilizer)

The stabilizer is not particularly limited so long as it does not adversely affect the pH sensitive carrier, and known stabilizers can be used, examples of which include saturated or unsaturated alcohols having 4 to 20 carbon atoms such as 1-octanol, 1-dodecanol, 1-hexadecanol, and 1-eicosanol; saturated or unsaturated fatty acids having 12 to 18 carbon atoms such as lauric acid, myristic acid, palmitic acid, stearic acid, and oleic acid; alkyl esters (alkyl having 1 to 3 carbon atoms) of saturated or unsaturated fatty acids having 8 to 18 carbon atoms such as methyl caprylate (methyl octanoate), ethyl caprylate (ethyl octanoate), methyl laurate, ethyl laurate, ethyl myristate, ethyl palmitate, ethyl stearate, methyl oleate, and ethyl oleate; D(L)-amino acids such as D(L)-alanine, arginine, asparagine, aspartic acid, cysteine, glutamine, glycine, histidine, leucine, isoleucine, lysine, methionine, proline, serine, threonine, tryptophan, tyrosine, valine, phenylalanine; amino acid triglycerides such as tricaproin and tricaprylin; polyoxyethylene sorbitan trifatty acid esters having 12 to 18 carbon atoms such as polyoxyethylene sorbitan tripalmitic acid ester and polyoxyethylene sorbitan trioleic acid ester (for example, Tween 65 and Tween 85); polyoxyethylene alkyl esters having 12 to 18 carbon atoms such as polyoxyethylene lauric acid ester, polyoxyethylene myristic acid ester, polyoxyethylene palmitic acid ester, and polyoxyethylene stearic acid ester (for example, PEG20 stearyl ether and PEG23 lauryl ether); polyoxyalkylene hardened castor oil (for example, PEG10 hardened castor oil, PEG40 hardened castor oil, and PEG60 hardened castor oil); saturated or unsaturated monofatty acid glycerol ester having 8 to 18 carbon atoms such as caprylin (glycerol octanoate), glycerol monocaprate, glycerol monolaurate, glycerol monomyristate, glycerol monopalmitate, glycerol monostearate, and glycerol monooleate; difatty acid glycerol having 8 to 16 carbon atoms such as glycerol dioctanoate, glycerol dicaprate, glycerol dilaurate, glycerol dimyristate ester, and glycerol dipalmitate; $\alpha$-tocopherol acetic acid ester, castor oil, soybean oil, cholesterol, squalene, squalane, lactose, ascorbyl palmitate, benzyl benzoate, methyl paraoxybenzoate, ethyl paraoxybenzoate, propyl paraoxybenzoate, butyl paraoxybenzoate, and the like. Incidentally, the "number of carbon atoms" in the stabilizer means the number of carbon atoms in the fatty acid component (acyl group) constituting the hydrophobic moiety.

The method for adding an additive such as a stabilizer is not particularly limited. For example, the additive may be added to an aqueous solution containing the pH sensitive compound or an aqueous solution containing the amphiphilic substance, or at the time of preparing a thin film, the additive may be dissolved together with the constituent components of the pH sensitive carrier, and by using a thin film containing these components, an aqueous solution containing the pH sensitive carrier may be obtained.

[Step 2: mixed liquid preparation step]

In this step, the pH sensitive carrier obtained in step 1 described above is mixed with a physiologically active substance, and thereby a mixed liquid is prepared.

Regarding the method of mixing a pH sensitive carrier and a physiologically active substance, any known method can be selected as appropriate according to the type of the physiologically active substance. This method is not particularly limited; however, for example, a method of mixing a pH sensitive carrier and a physiologically active substance into a medium such as an aqueous solvent or an excipient may be mentioned. For example, the physiologically active substance may be added to a dispersion liquid of the pH sensitive carrier obtained in step 1. At that time, the physiologically active substance may be added directly, or may be dissolved or dispersed in a solvent in advance and then added. Here, regarding the solvent for dissolving or dispersing the physiologically active substance, for example, the solvent mentioned in step 1 described above can be used.

The concentration of the pH sensitive carrier in the mixed liquid is preferably 50 nmol/mL or more, more preferably 100 nmol/mL or more, even more preferably 200 nmol/mL or more, and particularly preferably 500 nmol/mL or more. When the concentration is in the range described above, since the pH sensitive carrier forms a compact aggregate in the pH adjustment step that will be described below, the pH sensitive carrier can capture the physiologically active substance more satisfactorily. From the viewpoint of safety, the concentration of the pH sensitive carrier is preferably 1,000 nmol/mL or less.

A preferred content of the physiologically active substance in the mixed liquid is, for example, 0.001 to 10 mg with respect to 1,000 nmol of the amphiphilic substance that constitutes the pH sensitive carrier. Meanwhile, a more suitable content can be selected as appropriate according to the type of the physiologically active substance.

In this step, a substance having stimuli that activate natural immunity that will be described below, or known additives may be further added to the mixed liquid. That is, the complex of the present invention can also contain these substances.

[Step 3: pH adjustment step]

In this step, the pH of the mixed liquid obtained in step 2 is adjusted to a value of less than 6.4. Thereby, the pH sensitive carrier can form an aggregate while incorporating the physiologically active substance.

The method of adjusting the pH is not particularly limited, and a method of adding a known acid such as hydrochloric acid, sulfuric acid, or nitric acid into the mixed liquid may be mentioned. The amount of addition of the acid can be appropriately regulated by the target pH value.

The target pH for this step is not particularly limited as long as the value is lower than 6.4; however, from the viewpoint that the crosslinked structure of the aggregate becomes more compact and can capture the physiologically active substance more efficiently, it is more preferable as the pH is lower. However, in a case in which the pH is excessively low, there is a risk that the physiologically active substance may be denatured. Therefore, in this step, it is preferable that the pH of the solution is adjusted to a value of from 2.0 to 5.0, and more preferably adjusted to a value of from 2.0 to 4.0.

By adjusting the mixed liquid to a desired acidic pH and then leaving the mixed liquid to stand, a more compact aggregate of the pH sensitive carrier can be obtained. At this time, the lower limit of the standing time is not particularly limited; however, the lower limit is preferably 10 minutes or more, more preferably 30 minutes or more, and even more preferably 1 hour or more. When the standing time is in the range described above, since a compact aggregate is formed, the physiologically active substance can be retained satisfactorily within the aggregate. Furthermore, the upper limit of the standing time is not particularly limited. The standing temperature is also not particularly limited; however, the standing temperature is preferably room temperature (25°C) to about 40°C.

Through the steps 1 to 3 described above, a complex in which an aggregate of the pH sensitive carrier and a physiologically active substance are integrated can be obtained. After step 3, the complex may be isolated by removing the solvent by known means such as filtration.

When a liquid including the complex obtained as described above is treated with a filter (for example, having a pore size of 0.22 μm), and the concentrations of the physiologically active substance before and after a filtration treatment are measured, the integration ratio of the physiologically active substance (proportion of the amount of the physiologically active substance incorporated into an aggregate of the pH sensitive carrier with respect to the total amount of the physiologically active substance added) can be checked. Here, the concentration of the physiologically active substance can be measured by any known method. Meanwhile, in the present specification, the integration efficiency of the physiologically active substance is defined as a value calculated by the following formula 3 based on the measurement of the absorbance before and after a filtration treatment. At this time, the absorbance may be measured at a wavelength corresponding to the absorption wavelength range of the physiologically active substance (for example, 280 nm in the case of a protein or a peptide, and 260 nm in the case of a nucleic acid). Alternatively, the liquid is colored using a commercially available quantification kit, and then the absorbance may be measured at a wavelength that is recommended by the protocol of the kit.

[Math. 2]

```
(Formula 3)
Integration   efficiency   of   physiologically   active
substance (%) = [(Absorbance before filtration treatment
-  Absorbance  after  filtration  treatment)/(Absorbance
before filtration treatment)] × 100
```

<Use applications>

The complex according to the present invention can be used for the treatment of a subject as the complex alone or as a composition formed by mixing with other components (hereinafter, also referred to as pH sensitive composition).

The pH sensitive composition may be in the form of a solid preparation such as a tablet, a powder, or a capsule; however, it is preferable that the pH sensitive composition is in the form of a liquid preparation such as an injectable preparation. The liquid preparation may be supplied as a dried product that is reproduced with water or another appropriate excipient at the time of use.

It is desirable that the tablet and the capsule are subjected to enteric coating by a conventional method. Regarding the enteric coating, any enteric coating that is conventionally used in the pertinent field can be utilized. Furthermore, the capsule may contain either a powder or a liquid.

[Other components]

Examples of the other components that can be included in the pH sensitive composition include a substance having stimuli that activate natural immunity, pharmaceutical additives, and the like.

(Substance having stimuli that activate natural immunity)

A substance having stimuli that activate natural immunity means a substance which is recognized by a receptor for structural pattern recognition and leads immunocompetent cells into an active state. Meanwhile, this substance may overlap with the physiologically active substance described above.

The substance having stimuli that activate natural immunity is not particularly limited; however, the substance is preferably an agonist with respect to a Toll-like receptor.

Specific examples of the substance having stimuli that activate natural immunity include, but are not particularly limited to, mineral salts such as alum; gel type adjuvants such as aluminum hydroxide, aluminum phosphate, and calcium phosphate; microbial adjuvants such as an immunoregulatory DNA sequence including a CpG motif, an immunostimulatory RNA molecule, endotoxins (lipopolysaccharide (LPS; endotoxin) and monophosphoryl lipid A (MPL: registered trademark)), exotoxins (cholera toxin, E. coli heat-labile toxin, and pertussis toxin), muramyl dipeptide, and flagellin; oily adjuvants such as incomplete Freund's adjuvant (IFA), oily adjuvant biodegradable microspheres for liquid paraffin, lanolin, and the like, saponin (QS-21, Quil-A, and the like), synthetic adjuvants such as a nonionic block copolymer, muramyl peptide analogues, polyphosphazen, synthetic polynucleotides (a non-CpG synthetic polynucleotide and the like), and imidazoquinoline; cationic lipids such as DOTAP, DC-Chol, and DDA; single-stranded RNA; doublestranded RNA; and the like. Among them, from the viewpoint of safety, aluminum compounds such as aluminum hydroxide and aluminum phosphate are preferred. That is, a preferred embodiment of the present invention is a pH sensitive composition including the above-described complex and an aluminum compound.

The substances having stimuli that activate natural immunity may be used singly, or two or more kinds thereof may be used in combination.

The content of the substance having stimuli that activate natural immunity may vary depending on the type of the substance; however, the content is preferably 0.0227 to 1,280 mol, more preferably 0.0227 to 22.7 mol, and even more preferably 0.2 to 2.27 mol, with respect to 100 mol of the amphiphilic substance that constitutes the complex. When the content of the substance having stimuli that activate natural immunity is 0.0227 mol or more, immunoresponse can be suitably induced, and therefore, it is preferable. On the other hand, when the content of the substance having stimuli that activate natural immunity is 1, 280 mol or less, it is preferable because the cost can be reduced. Meanwhile, in a case in which an aluminum compound is used as the substance having stimuli that activate natural immunity, the content refers to the content of the aluminum element.

(Pharmaceutical additives)

In a case in which the pH sensitive composition is a liquid preparation, the pharmaceutical additives can include a solvent (for example, physiological saline, sterile water, or a buffer solution), a membrane stabilizer (for example, cholesterol), an isotonizing agent (for example, sodium chloride, glucose, or glycerin), an antioxidant (for example, tocopherol, ascorbic acid, or glutathione), an antiseptic agent (for example, chlorobutanol or paraben), and the like. The above-described solvent may be a solvent that is used at the time of producing the complex. At that time, in order to prevent that aggregation of the pH sensitive carrier is dissociated, and thereby the physiologically active substance is released, the pH of the solvent to be used is preferably less than 7.0, and more preferably less than 6.4.

In a case in which the pH sensitive composition is a solid preparation, the pharmaceutical additives can include an excipient (for example, sugars such as lactose and sucrose, starches such as corn starch, celluloses such as crystalline cellulose, gum arabic, magnesium metasilicate aluminate, or calcium phosphate), a lubricating agent (for example, magnesium stearate, talc, or polyethylene glycol), a binder (for example, sugars such as mannitol and sucrose, crystalline cellulose, polyvinylpyrrolidone, or hydroxypropylmethyl cellulose), a disintegrant (for example, starches such as potato starch, celluloses such as carboxymethyl cellulose, or crosslinked polyvinylpyrrolidone), a coloring agent, a corrigent, and the like.

[Therapeutic method]

The administration form employed in a case in which the complex of the present invention or a pH sensitive composition containing this complex is used for the treatment of a subject, is not particularly limited, and examples include oral administration, and parenteral administration such as intravenous injection, intraarterial injection, subcutaneous injection, intradermal injection, intramuscular injection, intrathecal injection, percutaneous administration, or transdermal absorption. For example, in the case of using a peptide and a protein as the physiologically active substances, parenteral administration, particularly administration by subcutaneous injection, intradermal injection, intramuscular injection, or intravenous injection, is preferred.

When the complex of the present invention or a pH sensitive composition including this is administered, mechanical stirring using pipetting, a homogenizer, or the like may be carried out in advance so that the complex passes through a syringe needle and is delivered to a desired site.

When the complex of the present invention or a pH sensitive composition including this is administered to a subject, and the external environment of the complex or the pH sensitive composition including this acquires a pH value lower than a physiological pH (for example, less than pH 6.4), the complex or the pH sensitive composition exhibits a membrane disruptive function promoting effect, or a membrane disruptive function promoting effect and a membrane fusion function promoting effect, and can release the physiologically active substance specifically and efficiently.

Accordingly, a method for treating or preventing a disease, the method including administering an effective amount of the above-described complex or a pH sensitive composition including the above-described complex perorally or parenterally to a subject in need of treatment or prevention is provided by the present invention.

The subject mentioned above is preferably a mammal, and particularly preferably a human.

Examples of the disease include cancers such as prostate cancer, lung cancer, colon cancer, kidney cancer, stomach cancer, brain tumor, and breast cancer; infectious diseases such as HIV (Human Immunodeficiency Virus), hepatitis C, and hepatitis B; and central nervous diseases such as Alzheimer's disease and Parkinson's disease.

That is, according to a preferred embodiment of the present invention, a method for treating or preventing a disease is provided. Regarding such findings, the technical common knowledge at the time of filing can be referred to as appropriate.

Furthermore, according to an embodiment of the present invention, the complex of the present invention may also directly transport a physiologically active substance to cells by culture. That is, according to an embodiment of the present invention, a culture method for transporting a physiologically active substance to cells is provided.

The culture method includes a step of culturing cells in a medium containing the complex of the present invention.

The medium is not particularly limited, and any known medium can be used. Specific examples include MEM, DMEM, and RPMI.

The amount of addition of the complex to the medium is not particularly limited; however, the total molar concentration of the pH sensitive compound and the amphiphilic substance is preferably 0.73 µmol/L to 7.4 mmol/L, more preferably 7.3 µmol/L to 6.5 mmol/L, and even more preferably 8.0 µmol/L to 4.2 mmol/L.

Furthermore, the pH of the medium is preferably 8.0 to 6.5, from the viewpoint of preventing destabilization of the pH sensitive compound while retaining the form of the complex.

The cell to which the physiologically active substance is transported is not particularly limited; however, examples include cells collected from the subject, and cultured cells that have established a strain.

At this time, specific examples of the cells collected from the subject or the cultured cells that have established a strain include dendritic cells, NK (Natural Killer) cells, T-lymphocytes, B-lymphocytes, lymphocytic cells, and the like.

Among the cells described above, it is preferable to use cells collected from a subject, and it is more preferable to use dendritic cells, NK cells, T-cells, lymphocytic cells, and the like collected from a subject.

In the case of using cells collected from a subject, the cells can be collected by subjecting the subject to blood collection, biopsy, or the like. That is, according to an embodiment, the culture method can include a step of collecting cells from a subject.

Meanwhile, the culture cells may be administered to the subject. Thereby, treatment or prevention of a disease of the subject can be achieved. That is, according to an embodiment of the present invention, a method for treating or preventing a disease is provided.

According to a preferred embodiment, the method for treating or preventing a disease includes a step of collecting cells from a subject; a step of culturing the cells thus collected in a medium including the complex of the present invention; and a step of administering the cultured cells to the subject.

Thereby, treatment or prevention of a disease can be achieved. Meanwhile, specific examples of the disease include those mentioned above.

Furthermore, the complex according to the present invention can maintain an aggregated state for a certain time even if the complex is exposed to a physiological pH environment. Therefore, the complex of the present invention is delivered to a desired site in vivo, subsequently stays at the site in a state of retaining an aggregated form, and can implement controlled release of the physiologically active substance. In other words, an aggregate formed as a pH sensitive carrier aggregates under acidic conditions can function as a controlled release device of a physiologically active substance. Therefore, a preferred embodiment of the present invention is a controlled release agent containing the above-described complex. The form of administration and dosage form are as described above.

Thereby, treatment or prevention of a disease can be achieved. Meanwhile, specific examples of the disease include those mentioned above.

Examples

The effects of the present invention will be described using the following Examples and Comparative Examples. In the Examples, description of the unit "parts" or "percent (%)" may be used; however, unless particularly stated otherwise, the unit represents "parts by weight" or "percent (%) by weight". Furthermore, unless particularly stated otherwise, the various operations were carried out at room temperature (25°C).

<Raw materials>

In the Examples, the following compounds were used. In a case in which the reagent name and the product name are the same, the product name will not be described.

[pH sensitive compound]

- Deoxycholic acid (PharmaGrade, manufactured by Sigma-Aldrich Corp.)
- Ursodeoxycholic acid (manufactured by Tokyo Chemical Industry Co., Ltd.)
- Chenodeoxycholic acid (manufactured by Tokyo Chemical Industry Co., Ltd.)
- Hyodeoxycholic acid (manufactured by Tokyo Chemical Industry Co., Ltd.)
- Cholic acid (manufactured by Tokyo Chemical Industry Co., Ltd.)
- Glycyrrhizic acid (manufactured by Tokyo Chemical Industry Co., Ltd.)
- Glycodeoxycholic acid (manufactured by Tokyo Chemical Industry Co., Ltd.)

[Amphiphilic substance]

- DLPC (1,2-dilauroyl-sn-glycero-3-phosphatidylcholine: manufactured by NOF CORPORATION, COATSOME MC-1212)
- DDPC (1,2-didecanoyl-sn-glycero-3-phosphatidylcholine: manufactured by NOF CORPORATION, COATSOME MC-1010)
- Polyoxyethylene sorbitan monofatty acid ester (Tween 20 (number of carbon atoms: 12), Tween 80 (number of carbon atoms: 18); manufactured by Tokyo Chemical Industry Co., Ltd.)
- Sorbitan fatty acid ester (SPAN 80 (number of carbon atoms: 18), manufactured by NACALAI TESQUE, INC.)
- Polyoxyethylene castor oil (PEG10 castor oil, polyoxyethylene 10 castor oil, manufactured by Wako Pure Chemical Industries, Ltd.)

[Physiologically active substance]

- Model protein: OVA (molecular weight 45 kDa, manufactured by Sigma-Aldrich Corp.)
- Model DNA: Salmon-derived DNA (molecular weight 500 to 1,000 bp, manufactured by Wako Pure Chemical Industries, Ltd.)
- Model peptide: SIINFEKL (synthesized on commission)
- Model peptide: RGPESRLL (synthesized on commission)

[Others]

- Hydrochloric acid
- Methanol (manufactured by NACALAI TESQUE, INC.)
- Chloroform (manufactured by Wako Pure Chemical Industries, Ltd.)
- Acetic acid (manufactured by Wako Pure Chemical Industries, Ltd.)
- Sodium acetate (manufactured by KANTO CHEMICAL CO., INC.)
- MES-Na (manufactured by Merck & Co., Inc.)
- Hepes-Na (manufactured by NACALAI TESQUE, INC.)
- Sodium chloride (manufactured by KANTO CHEMICAL CO., INC.)
- Pyranine (manufactured by Tokyo Chemical Industry Co., Ltd.)
- DPX (p-xylene-bis-pyridinium bromide: manufactured by Molecular Probes, Inc.)

- PBS (Phosphate Buffered Salts: manufactured by Takara Bio, Inc., PBS Tablets)
- Aqueous solution of sodium hydroxide (0.1 mol/L: manufactured by NACALAI TESQUE, INC.)
- Hydrochloric acid (0.1 mol/L, 1 mol/L: manufactured by NACALAI TESQUE, INC.)
- Triton-XlOO (manufactured by Wako Pure Chemical Industries, Ltd., TRITON X100)
- DMEM (manufactured by NACALAI TESQUE, INC., Dulbecco Modified Eagle's Medium)

<Preparation of pH sensitive carrier solution>

An amphiphilic substance dissolved in chloroform and a pH sensitive compound dissolved in methanol or chloroform were mixed in a 10-mL pear-shaped flask, and a thin film was produced using a rotary evaporator (BuCHI). Meanwhile, the ratio of the amphiphilic substance and the pH sensitive compound was adjusted to a desired ratio (molar ratio 100 : 10, 100 : 20, 100 : 640, or the like).

1 mL of a MES buffer (MES: 25 mM, NaCl: 125 mM, pH 7.4) was added to the thin film thus produced, the thin film was dispersed by irradiating the mixture for one minute at room temperature using an ultrasonic irradiation apparatus (USC-J), and thus a dispersion liquid of a pH sensitive carrier was obtained.

<Leaching test: measurement of Leakage (leaching rate)>

Leakage (leaching rate) was evaluated using EYPC liposomes encapsulating Pyranine as a fluorescent substance and DPX as a quenching agent, according to the method described in K. Kono et al., Bioconjugate Chem. 2008, 19, 1040-1048.

3,000 nmol of EYPC (egg yolk phosphatidylcholine) dissolved in chloroform was measured and introduced into a 10-mL pear-shaped flask, and a thin film was produced using a rotary evaporator (BuCHI). 500 $\mu$L of a Pyranine solution (Pyraine: 35 mM, DPX: 50 mM, MES: 25 mM, pH 7.4) was added to the thin film, and the thin film was dispersed using an ultrasonic irradiation apparatus (USC-J). Subsequently, the dispersion liquid was passed through a polycarbonate film having a pore diameter of 100 nm using an extruder, and thus the particle size was arranged. Replacement of the external aqueous layer was performed using MES buffer (MES: 25 mM, NaCl: 125 mM, pH 7.4) and a G100 column, and a dispersion liquid of EYPC liposome encapsulating a fluorescent substance was obtained. The concentration of phospholipids (choline group) was determined using PHOSPHOLIPID C-TEST WAKO (manufactured by Wako Pure Chemical Industries, Ltd.), and the concentration was adjusted using MES buffer (MES: 25 mM, NaCl: 125 mM, pH 7.4) so as to obtain a phospholipid concentration of 1.0 mmol/L.

20 $\mu$L of an EYPC liposome dispersion liquid having the concentration adjusted, and 20 $\mu$L of an evaluation sample dispersion liquid were introduced into 2,960 $\mu$L of MES buffer (MES: 25 mM, NaCl: 125 mM) adjusted to various pH values, and the mixtures were incubated for 90 or 30 minutes at 37°C (in the Examples, unless particularly described others, the results for 90 minutes are described). The Leakage was monitored by observing the fluorescence of Ex 416 nm and Em 512 nm using a spectrophotometer FP-6500. Measurement at pH 4.0 to pH 3.0 was performed using an acetic acid buffer (acetic acid: 25 mM, NaCl: 125 mM).

Meanwhile, the case of an EYPC liposome dispersion liquid only was designated as 0%, the value obtained in a case in which 30 $\mu$L of Triton-XlOO diluted to ten time was added was designated as 100%, and the leakage was calculated.

Specifically, the leakage was calculated according to the following formula 4. In the following formula, the fluorescence intensity thus measured was designated as L, the fluorescence intensity of only the EYPC liposome dispersion liquid encapsulating a fluorescent substance was designated as $L_0$, and the fluorescence intensity in the case of having Triton-X100 added thereto was designated as $L_{100}$.

[Math. 3]

$$\text{(Formula 4)}$$

$$\text{Leakage (\%)} = (L - L_0)/(L_{100} - L_0) \times 100$$

<Experiment 1. Functional evaluation for aggregate of pH sensitive carrier>

The membrane disruptive function promoting effect of an aggregate of a pH sensitive carrier was evaluated. Regarding a dispersion liquid of the pH sensitive carrier, a dispersion liquid containing 1,000 nmol of DLPC and 1,600 nmol of deoxycholic acid in 1 mL was used. The pH was adjusted using 1 N sulfuric acid.

The pH of the dispersion liquid of the pH sensitive carrier was set to various values, and a precipitate was generated along with a decrease in pH (Fig. 1A). The precipitate was generated immediately after lowering of the pH and exhibited a behavior of being reduced from a gel-like state with the passage of time (Fig. 1B). It is speculated that in an environment at low pH, the pH sensitive carriers are fused with one another and formed a precipitate. Hereinafter, this precipitate will also be referred to as "aggregate of the pH sensitive carrier". The membrane disruptive function promoting effect of an aggregate of the pH sensitive carrier generated at various pH values was measured, and surprisingly, all of the aggregates of the pH sensitive carrier had the membrane disruptive function promoting effect. It has become clear that the pH sensitive carrier has a membrane disruptive function promoting effect, and maintains the effect even if the pH sensitive carrier forms an aggregate (Figs. 1C and 1D). Meanwhile, the data corresponding to Fig. 1C are shown in the following Table 1, and the data corresponding to Fig. 1D are shown in the following Table 2.

[Table 1]

(Table 1)

| pH | 7.2 | 7.1 | 6.96 | 6.72 | 6.5 | 6.3 | 6.02 | 5.64 | 3.64 | 3.14 | 2.02 | 1 |
|---|---|---|---|---|---|---|---|---|---|---|---|---|
| pH 7.4 | 4.114 | 4.064 | 4.049 | 4.483 | 4.602 | 3.673 | 4.386 | 3.993 | 4.340 | 3.966 | 4.422 | 4.737 |
| pH 5.0 | 66.496 | 71.453 | 76.806 | 64.259 | 69.399 | 67.442 | 67.254 | 66.667 | 68.020 | 70.337 | 72.842 | 69.966 |

[Table 2]

(Table 2)

|  | DLPC alone | Deoxycholic acid alone |
|---|---|---|
| pH 7.4 | 1.188 | 0.980 |
| pH 5.0 | 2.587 | 15.559 |

<Experiment 2. Integration of aggregate of pH sensitive carrier and protein>

It was expected that when a pH sensitive carrier forms an aggregate, a physiologically active substance is incorporated therein, and a complex is formed thereby. Thus, first, verification of an evaluation system was carried out. OVA was used as a protein, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH was adjusted using 0.1 N hydrochloric acid.

1. First, OVA was mixed with the dispersion liquid of the pH sensitive carrier to prepare a mixed liquid, subsequently hydrochloric acid was added thereto, and the pH of the mixed liquid was decreased from 7.4 to 4.0. Furthermore, the mixed liquid was left to stand for about 2 hours and then was passed through a 0.22-$\mu$m filter (SLGV033RS manufactured by Merck Millipore GmbH). The solution including a precipitate acquire a transparent appearance through filtering, and separation of an aggregate of the pH sensitive carrier was suggested (Fig. 2A). Furthermore, the solution before and after the filtration treatment was colored using PHOSPHOLIPID C-TEST WAKO (manufactured by Wako Pure Chemical Industries, Ltd.), and measurement of the absorbance at a wavelength of 600 nm was carried out. While the absorbance before filtration was 0.295 (corresponding to a phospholipid concentration of 1.5 mg/mL), the absorbance after filtration was 0.001 (corresponding to a phospholipid concentration of 0.0 mg/mL) (Fig. 2B). It was obvious that the aggregate of the pH sensitive carrier can be almost completely separated by this filtration.
2. Next, separation of the protein was checked. PBS at pH 7.4 containing OVA was passed through a 0.22-$\mu$m filter similarly to the previous operation. The liquid before and after the filtration treatment was colored using a protein quantification kit (protein assay CBB solution, manufactured by NACALAI TESQUE, INC.), and measurement of the absorbance at a wavelength of 595 nm was performed. The absorbance before filtration was 0.616 while the absorbance after filtration 0.601, and thus the two exhibited similar values (Fig. 3A). Meanwhile, the absorbance was converted to concentration using a calibration curve, and while the OVA concentration before filtration was 0.81 mg/mL, the OVA concentration after filtration was 0.79 mg/mL. The concentrations thus obtained were consistent with the feed concentration (Fig. 3B). It was confirmed that free OVA all escaped without being captured by the filtration. Therefore, it was considered that integration of the aggregate of the pH sensitive carrier and the protein can be evaluated by measuring the protein concentrations before and after passage through a filter (pore diameter 0.22 $\mu$m).
3. Subsequently, integration of a protein and the pH sensitive carrier was evaluated. OVA was used as the protein, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH was adjusted using 0.1 N hydrochloric acid. The time for standing was set to about 2 hours.

OVA was mixed with a dispersion liquid of the pH sensitive carrier, and a mixed liquid was prepared. Subsequently, the mixed liquid was passed through a filter by a test method similar to section 2. of Experiment 2, and the change in the absorbance (corresponding to the OVA concentration) before and after a filtration treatment was investigated. In the case of a neutral pH, the absorbance before filtration was 0.59, and the absorbance after filtration was 0.58. The absorbance did not change before and after the passage through a filter, and it was suggested that the pH sensitive carrier was not integrated with OVA (Fig. 4A). On the other hand, in a case in which the pH was adjusted to pH 3.5, the absorbance before filtration was 0.57, while the absorbance after filtration was 0.011 and this value was a very small value compared to the value before filtration (Fig. 4B). From these results, it was suggested that by lowering the pH of the pH sensitive carrier containing a protein, a complex of an aggregate of the pH sensitive carrier and the protein is obtained.

<Experiment 3. Examination of pH enabling integration>

The range of pH that enables integration between a protein and an aggregate of the pH sensitive carrier was investigated. OVA was used as the protein, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH was adjusted using 0.1 N hydrochloric acid. The time for standing was set to 1 hour, and to 1 day.
OVA was mixed with a dispersion liquid of the pH sensitive carrier to prepare a mixed liquid, and the mixed liquid was set to various pH values. The mixed liquid was left to stand for one hour or one day, subsequently measurement of the absorbance before and after a filtration treatment by a method similar to that of section 2. of Experiment 2, and thus an evaluation of integration was performed. As a result, in a case in which the pH was adjusted to 7.4, 6.8, or 6.4, the absorbance almost did not change before and after filtration (Figs. 5A to 5C). On the other hand, in a case in which the pH was adjusted to 5.9 to 1.9, the absorbance after filtration had smaller values compared to the values obtained before filtration (Figs. 5A to 5C). It was suggested that integration of an aggregate of the pH sensitive carrier and a protein is enabled in this pH range (pH 5.9 to 1.9).
Meanwhile, the variation in the absorbance before and after a filtration treatment exhibited a similar tendency in a case in which the mixed liquid was left to stand for 1 hour (Fig. 5B) and in a case in which the mixed liquid was left to stand for one day (Fig. 5C). It became clear that the range of pH that enabled integration is not dependent on the time for standing. Meanwhile, the data corresponding to Fig. 5B are shown in the following Table 3, and the data corresponding to Fig. 5C are shown in the following Table 4.

[Table 3]

(Table 3)

| pH | Before filtration | After filtration |
|------|-------------------|------------------|
| 7.38 | 0.621 | 0.584 |
| 6.84 | 0.621 | 0.632 |
| 6.4 | 0.610 | 0.585 |
| 5.85 | 0.556 | 0.432 |
| 3.8 | 0.541 | 0.247 |
| 2.58 | 0.645 | 0.088 |
| 2.2 | 0.555 | 0.062 |
| 2.05 | 0.556 | 0.065 |
| 1.91 | 0.552 | 0.062 |

[Table 4]

(Table 4)

| pH | Before filtration | After filtration |
|------|-------------------|------------------|
| 7.38 | 0.625 | 0.618 |
| 6.84 | 0.654 | 0.642 |
| 6.4 | 0.626 | 0.588 |
| 5.85 | 0.557 | 0.461 |
| 3.8 | 0.545 | 0.314 |
| 2.58 | 0.55 | 0.166 |
| 2.2 | 0.542 | 0.12 |

(continued)

| pH | Before filtration | After filtration |
|---|---|---|
| 2.05 | 0.555 | 0.081 |
| 1.91 | 0.536 | 0.041 |

<Experiment 4. Examination of amount of protein capable of integration>

Furthermore, an operation of the integration with OVA at various concentrations was carried out, and the amount of protein capable of integration was determined. The concentration of OVA was set to 0.001 mg/mL to 10 mg/mL. Regarding the dispersion liquid of the pH sensitive carrier, a dispersion liquid containing 1,000 nmol of DLPC and 1,600 nmol of deoxycholic acid in 1 mL was used. The pH was adjusted to 2 using 1 N sulfuric acid. The time for standing was set to 1 hour. Furthermore, measurement of the absorbance before and after a filtration treatment was carried out similarly to the method of section 2. of Experiment 2, and the absorbance thus obtained was converted to the OVA concentration.

As a result, with respect to the dispersion liquid of the pH sensitive carrier, which was 1,000 nmol/mL of DLPC, a decrease in the OVA concentration due to a filtration treatment was confirmed in the range of OVA concentration of 0.001 to 10 mg/mL (Fig. 6). Therefore, it was suggested that integration of the aggregate of the pH sensitive carrier and the protein is enabled in the above-described concentration range. Meanwhile, the data corresponding to Fig. 6 are shown in the following Table 5.

[Table 5]

(Table 5)

| OVA (mg/mL) | 0.001 | 0.01 | 0.1 | 1 | 10 |
|---|---|---|---|---|---|
| Before filtration | 0.085 | 0.097 | 0.185 | 0.759 | 1.667 |
| After filtration | 0.021 | 0.031 | 0.034 | 0.042 | 0.058 |

<5. Examination of concentration of pH sensitive carrier enabling integration>

Next, the concentration of the pH sensitive carrier that enables integration with a protein was investigated. OVA was used as the protein, and the concentration was adjusted to 0.8 mg/mL. Regarding the dispersion liquid of the pH sensitive carrier, a dispersion liquid containing 1,600 nmol of deoxycholic acid with respect to 1,000 nmol of DLPC in 1 mL was used. The pH was adjusted to 4 using 0.1 N hydrochloric acid. The time for standing was set to 1 hour.

Dispersion liquids of the pH sensitive carrier at various DLPC concentrations were prepared, measurement of the absorbance before and after a filtration treatment was carried out by a method similar to that of section 2. of Experiment 2, and integration with a protein was evaluated. In a case in which a dispersion liquid of the pH sensitive carrier at a low DLPC concentration was used, the change in the absorbance before and after a filtration treatment tended to be smaller (Fig. 7A). It is speculated that the amount of protein integrated has decreased. It is speculated that the dispersion liquid of the pH sensitive carrier at a low DLPC concentration formed a low-density aggregate, and the above-described results were obtained (Fig. 7B).

However, in the range of the concentration of the pH sensitive carrier of 1,000 to 50 nmol/mL of DLPC, in which the evaluation was performed, the absorbance after filtration had a smaller value compared to the absorbance before filtration, and results suggesting integration of the aggregate of the pH sensitive carrier and the protein were obtained. Therefore, it is speculated that the aggregate of the pH sensitive carrier and the protein formed a complex at the concentration in the above-described range. Meanwhile, the data corresponding to Fig. 7A are shown in the following Table 6.

[Table 6]

(Table 6)

| DLPC (nmol/mL) | 1000 | 200 | 100 | 50 |
|---|---|---|---|---|
| Before filtration | 0.567 | 0.539 | 0.530 | 0.554 |
| After filtration | 0.180 | 0.267 | 0.316 | 0.426 |

<Experiment 6. Influence exerted by mixing amount of deoxycholic acid on integration>

pH sensitive carriers having different mixing amounts of deoxycholic acid were prepared, and the influence exerted on the integration of a protein was investigated.

OVA was used as the protein, and the concentration was adjusted to 0.8 mg/mL. Regarding the dispersion liquid of the pH sensitive carrier, dispersion liquids containing various amounts of deoxycholic acid with respect to 1,000 nmol of DLPC in 1 mL were used. The pH was adjusted to 4 using 0.1 N hydrochloric acid. The time for standing was set to 1 hour. Furthermore, measurement of the absorbance before and after a filtration treatment was carried out by a method similar to that of section 2. of Experiment 2.

As a result of the evaluation, in a case in which the mixing amount of deoxycholic acid was a small amount, the change in the absorbance before and after filtration tended to become smaller (Fig. 8). It was suggested that the amount of the protein to be integrated was a small amount.

However, in the mixing amount of deoxycholic acid of 400 to 6,400 nmol/mL, at which the evaluation was performed, the absorbance after filtration was lower than the absorbance before filtration, and the results suggested integration of the carrier of the pH sensitive carrier and the protein. Therefore, it is speculated that the aggregate of the pH sensitive carrier and the protein were integrated in the mixing amount of deoxycholic acid described above. Above all, the integration efficiency for OVA based on the change in the absorbance was about 42% at 400 nmol/mL, about 60% at 1,600 nmol/mL, and about 64% at 6,400 nmol/mL. Thus, it was suggested that as the mixing amount of deoxycholic acid was larger, the integration efficiency for the protein was excellent. Meanwhile, the data corresponding to Fig. 8 are shown in the following Table 7.

[Table 7]

(Table 7)

| Deoxycholic acid (nmol/mL) | 400 | 1600 | 6400 |
|---|---|---|---|
| Before filtration | 0.502 | 0.564 | 0.527 |
| After filtration | 0.189 | 0.224 | 0.308 |

<Experiment 7. Membrane disruptive function promoting effect of complex>

The membrane disruptive function promoting effect of the complex in which a protein was supported on the aggregate of the pH sensitive carrier was evaluated. OVA was used as the protein, and the concentration was set to 0 to 10 mg/mL. Regarding the dispersion liquid of the pH sensitive carrier, a dispersion liquid containing 1,000 nmol of DLPC and 1,600 nmol of deoxycholic acid in 1 mL was used. The pH was adjusted to 3.5 using 1 N sulfuric acid. The time for standing was set to 1 hour.

A leaching test was performed by the above-described method, and as the amount of OVA to be integrated increased, the leakage at pH 5.0 tended to slightly decrease. However, at any amount of OVA, the complex satisfied the definition of the membrane disruptive function promoting effect (Fig. 9A). It became clear that a complex formed by supporting a protein on an aggregate of the pH sensitive carrier has a membrane disruptive function promoting effect.

Furthermore, the membrane disruptive function promoting effect of the complex in the presence of aluminum hydroxide was investigated. OVA was used as the protein, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH at the time of preparing an aggregate of the pH sensitive carrier was adjusted to 1.0 to 3.5 using 1 N sulfuric acid. The time for standing was set to 1 hour. Meanwhile, aluminum hydroxide was added to the pH sensitive carrier, subsequently the protein was added thereto, and finally the pH was adjusted. The final concentration of aluminum hydroxide was set to 1.0 mg/mL. That is, the amount of addition of aluminum element was 1,280 mol with respect to 100 mol of DLPC.

A leaching test was performed by the method described above, and the complex adjusted to any of the pH values exhibited a leakage similar to that of conventional pH sensitive carrier, in the presence of aluminum hydroxide (Alum) (Fig. 9B). It became clear that the membrane disruptive function promoting effect of the complex was not affected by aluminum hydroxide. Meanwhile, the data corresponding to Fig. 9A are shown in the following Table 8, and the data corresponding to Fig. 9B are shown in the following Table 9.

[Table 8]

(Table 8)

|  | DLPC alone | Deoxycholic acid alone | Complex OVA amount (mg/mL) | | | | | |
|---|---|---|---|---|---|---|---|---|
|  |  |  | 0 | 0.001 | 0.01 | 0.1 | 1 | 10 |
| pH 7.4 | 2.688 | 2.037 | 5.217 | 4.759 | 4.393 | 5.621 | 4.134 | 5.123 |
| pH 5.0 | 4.587 | 16.231 | 72.240 | 62.113 | 58.358 | 50.953 | 40.820 | 46.789 |

[Table 9]

(Table 9)

|  | pH 1.0 | pH 1.3 | pH 3.5 | normal micelle |
|---|---|---|---|---|
| pH 7.4 | 2.542 | 4.474 | 4.314 | 4.524 |
| pH 5.0 | 67.002 | 65.428 | 70.205 | 62.410 |

<Experiment 8. Influence exerted by amphiphilic substance and pH sensitive compound of pH sensitive carrier on integration of protein>

First, pH sensitive carriers formed by using various amphiphilic substances were used to attempt integration with a protein. OVA was used as the protein, and the concentration was adjusted to 0.8 mg/mL. Regarding the dispersion liquid of the pH sensitive carrier, a dispersion liquid containing 1,000 nmol of an amphiphilic substance and 1,600 nmol of deoxycholic acid in 1 mL was used. The pH at the time of preparing an aggregate of the pH sensitive carrier was adjusted to 4 using 1 N sulfuric acid. The time for standing was set to 1 hour. Measurement of the absorbance before and after a filtration treatment was carried out by a method similar to that of section 2. of Experiment 2.

In the case of OVA only, the absorbance almost did not change before and after a filtration treatment. On the other hand, in the case of pH sensitive carriers using DDPC, Tween 20, Tween 80, SPAN 80, and PEG 10 castor oil, the absorbance after filtration showed a very small value compared to the value before filtration (Fig. 10A). These results suggested integration of each of the aggregates of the pH sensitive carriers formed using various amphiphilic substances, and OVA. It is thought that the type of the amphiphilic substance does not have large influence on the integration of an aggregate of the pH sensitive carrier and a protein.

Subsequently, pH sensitive carriers formed using various pH sensitive compounds were used, and integration thereof with a protein was investigated. OVA was used as the protein, and the concentration was adjusted to 0.8 mg/mL. Regarding the dispersion liquid of the pH sensitive carrier, dispersion liquids including 1,000 nmol of DLPC and 1,600 nmol of various pH sensitive compounds in 1 mL were used. The pH at the time of preparing an aggregate of the pH sensitive carrier was adjusted to 4 using 1 N sulfuric acid. The time for standing was set to 1 hour. Furthermore, measurement of the absorbance before and after a filtration treatment was carried out by a method similar to that of section 2. of Experiment 2.

In the case of OVA only and in the case of deoxycholic acid only, change in the absorbance almost did not occur before and after a passage through a filter. On the other hand, in the cases of the pH sensitive carriers formed using ursodeoxycholic acid, chenodeoxycholic acid, hyodeoxycholic acid, cholic acid, glycyrrhizic acid, and glycodeoxycholic acid, the absorbance after filtration showed a very small value compared to the absorbance before filtration (Fig. 10B). These results suggested integration of aggregates of pH sensitive carriers formed using various pH sensitive compounds, and OVA. It is thought that the type of the pH sensitive compound does not have large influence on the integration of an aggregate of the pH sensitive carrier and a protein. Meanwhile, the data corresponding to Fig. 10A are shown in the following Table 10, and the data corresponding to Fig. 10B are shown in the following Table 11.

[Table 10]

(Table 10)

|  | Before filtration | After filtration |
|---|---|---|
| OVA only | 0.661 | 0.651 |
| DDPC-deoxycholic acid | 0.722 | 0.017 |
| Tween 20-deoxycholic acid | 0.674 | 0.020 |
| Tween 80-deoxycholic acid | 0.721 | 0.108 |
| Span 80-deoxycholic acid | 0.602 | 0.035 |
| Peg 10 castor oil-deoxycholic acid | 0.725 | 0.020 |

[Table 11]

(Table 11)

|  | Before filtration | After filtration |
|---|---|---|
| OVA only | 0.588 | 0.533 |
| Deoxycholic acid only | 0.584 | 0.569 |
| DLPC-ursodeoxycholic acid | 0.741 | 0.040 |
| DLPC-chenodeoxycholic acid | 0.720 | 0.036 |
| DLPC-hyodeoxycholic acid | 0.641 | 0.032 |
| DLPC-cholic acid | 0.605 | 0.050 |
| DLPC-glycyrrhizic acid | 0.697 | 0.034 |
| DLPC-glycodeoxycholic acid | 0.558 | 0.029 |

<Experiment 9. Integration of aggregate of pH sensitive carrier and nucleic acid>

With regard to physiologically active substances other than protein, it is also expected that the substances integrate with an aggregate of a pH sensitive carrier. Nucleic acid has attracted attention for realizing gene therapy and DNA vaccines, and nucleic acid is one of pharmaceutical candidates that require delivery to the cytosol. It is speculated that by integrating nucleic acid with an aggregate of a pH sensitive carrier, highly efficient endosome escape and delivery to the cytosol are achieved, and the integration can contributes to the materialization mentioned above. Thus, an investigation was conducted on the integration of an aggregate of a pH sensitive carrier and nucleic acid.

Salmon-derived deoxyribonucleic acid (DNA) was used as the nucleic acid, and the concentration was adjusted to 0.1 mg/mL. Regarding the dispersion liquid of the pH sensitive carrier, a dispersion liquid containing 1,000 nmol of DLPC and 1,600 nmol of deoxycholic acid in 1 mL was used. The pH at the time of preparing an aggregate of the pH sensitive carrier was adjusted to 4 using 1 N sulfuric acid. The mixed liquid was passed through a 0.22-$\mu$m filter, the absorbances at a wavelength of 260 nm of the liquid before and after the filtration treatment were measured, and thereby the deoxyribonucleic acid was detected. The time for standing was set to 2 hours.

In a case in which the pH sensitive carrier and deoxyribonucleic acid were mixed at a neutral pH, the absorbance was 2.0, and the mixture showed an absorbance similar to the absorbance obtained in the case of using deoxyribonucleic acid alone (Fig. 11). It was suggested that deoxyribonucleic acid and the pH sensitive carrier are not integrated at a neutral pH. On the other hand, in a case in which the pH was decrease, and an aggregate of the pH sensitive carrier was obtained, the absorbance after filtration was 0.92, and this value was about half the value obtained in the case of using deoxyribonucleic acid alone (Fig. 11). It was found that about half the amount of the deoxyribonucleic acid added was integrated with an aggregate of the pH sensitive carrier (Fig. 11). That is, the integration efficiency for deoxyribonucleic acid based on the change in absorbance was about 54%. Meanwhile, the data corresponding to Fig. 11 are shown in the following Table 12.

[Table 12]

(Table 12)

| PBS | 0.073 |
| --- | --- |
| DNA | 2.074 |
| DNA (after lowering of pH + filtration) | 2.068 |
| pH sensitive carrier + DNA | 1.992 |
| Aggregate of pH sensitive carrier + DNA (after filtration) | 0.918 |

<Experiment 10. Integration of aggregate of pH sensitive carrier and peptide>

Subsequently, integration of an aggregate of the pH sensitive carrier and a peptide was investigated. Peptides have been actively studied as candidates for promising pharmaceutical products such as antigens of cancer vaccines and intracellular signal transduction inhibitors. Since all of these require delivery to the cytosol, a synergistic effect with a pH sensitive carrier and an aggregate of a pH sensitive carrier is expected.

SIINFEKL and RGPESRLL were used as peptides, and the concentration of the peptide was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH at the time of preparing an aggregate of the pH sensitive carrier was adjusted to 4 using 1 N sulfuric acid. The time for standing was set to 2 hours. The mixed liquid was passed through a 0.22-$\mu$m filter, the liquid before and after filtration was colored using a peptide quantification kit (Lowry kit, manufactured by NACALAI TESQUE, INC.), and measurement of the absorbance at a wavelength of 750 nm was carried out.

With regard to the mixed liquid of the pH sensitive carrier and SIINFEKL peptide, the peptide-derived absorbance (coloration according to the Lowrry method) at a neutral pH was 0.44, and the mixed liquid showed an absorbance similar to the absorbance of the peptide alone. It was suggested that the pH sensitive carrier and the peptide were not integrated (Fig. 12A). On the other hand, in a case in which the pH was lowered to obtain an aggregate of the pH sensitive carrier, the absorbance of the solution after filtration was 0.32, and it became clear that the about 1/4 of the amount of the peptide was integrated with the aggregate of the pH sensitive carrier (Fig. 12A). That is, the integration efficiency of the SIINFEKL peptide based on the change in absorbance was about 27%. Furthermore, in the case of the RGPESRLL peptide, the above-mentioned values were 1.40 and 0.82, and it became clear that about 1/3 of the amount of the peptide was integrated with the aggregate of the pH sensitive carrier (Fig. 12B). That is, the integration efficiency of the RGPESRLL peptide based on the change in absorbance was about 41%.

It was found that a peptide having any of the sequences can integrate with an aggregate of the pH sensitive carrier. Meanwhile, the data corresponding to Fig. 12A are shown in the following Table 13, and the data corresponding to Fig. 12B are shown in the following Table 14.

[Table 13]

(Table 13)

| PBS | 0.069 |
| --- | --- |
| Peptide | 0.445 |
| Peptide (after lowering of pH + filtration) | 0.442 |
| pH sensitive carrier + peptide | 0.446 |
| Aggregate of pH sensitive carrier + peptide (after filtration) | 0.327 |

[Table 14]

(Table 14)

| | |
|---|---|
| PBS | 0.068 |
| Peptide | 1.397 |
| Peptide (after lowering of pH + filtration) | 1.429 |
| pH sensitive carrier + peptide | 1.382 |
| Aggregate of pH sensitive carrier + peptide (after filtration) | 0.820 |

<Experiment 11. State of aggregate of pH sensitive carrier at neutral pH, and state of integrated physiologically active substance>

The pH of blood or blood plasma is neutral. Therefore, when administration of an aggregate of a pH sensitive carrier to a living body is considered, it was contemplated that there is a need for investigating the state of the aggregate of the pH sensitive carrier in a neutral environment, and the state of an integrated physiologically active substance.

OVA was used as the physiologically active substance, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The pH was adjusted to 1 using 1 N sulfuric acid (Fig. 13C). Alternatively, the pH was adjusted to 2.6 using 0.1 N hydrochloric acid (Figs. 13A, 13B, and 13D). When the pH was adjusted to be neutral, 0.1 N sodium hydroxide was used. The time for standing was set to 2 hours.

A solution of the aggregate of the pH sensitive carrier was adjusted to a neutral pH, subsequently the solution was left to stand for one day, and a precipitate of the aggregate of the pH sensitive carrier disappeared (Figs. 13A and 13B). The solution thus obtained was evaluated in a leaching test, and the solution showed a leakage similar to that of a conventional pH sensitive carrier that was not subjected to an operation (described as normal (pH 7.4) in the diagram). Thus, it was confirmed that the solution had a membrane disruptive function promoting effect (Fig. 13C pH 7.4 → 1.15 → 7.4). Meanwhile, also in a case in which the pH was adjusted to a neutral pH or higher, similar results were obtained (Fig. 13C). It was suggested that when the aggregate of the pH sensitive carrier is transferred to a neutral pH or higher, the aggregates forming a precipitate are dissociated, and the pH sensitive carrier returns to a particulate state. Meanwhile, the data corresponding to Fig. 13C are shown in the following Table 15.

[Table 15]

(Table 15)

| | pH 7.4→ 1.15→7.4 | pH 7.4→ 1.15→11.5 | pH 7.4→ 1.23→11.3 | pH 7.4→ 1.0→11.3 | normal (pH 7.4) |
|---|---|---|---|---|---|
| pH 7.4 | 3.430 | 1.868 | 2.005 | 1.757 | 1.926 |
| pH 5.0 | 69.773 | 66.563 | 63.381 | 65.021 | 69.333 |

Subsequently, the state of integrated physiologically active substance was investigated. A solution of the aggregate of the pH sensitive carrier integrated with OVA was adjusted to a neutral pH, subsequently the solution was left to stand for one day, and thereby a precipitate disappeared. Similarly to section 2. of Experiment 2, the solution thus obtained was passed through a filter, and the amount of free OVA in the solution was evaluated. As a result, the absorbance thus obtained was 0.61 (Fig. 13D, adjusted to pH 7.3 and then filtered), and this showed a large value compared to the absorbance (0.10) in a case in which the solution was not adjusted to a neutral pH (Fig. 13D, pH 2.6 filtered). Furthermore, this value of absorbance was consistent with the absorbance (0.57) obtained before filtering the solution (Fig. 13D, pH 2.6 not filtered), and it was suggested that the integrated OVA was all in a state of being dissociated. It is speculated that by transferring the aggregate of the pH sensitive carrier to a neutral pH, the integrated physiologically active substance was dissociated and was released from the aggregate of the pH sensitive carrier.

<Experiment 12. Time for retaining aggregate state of pH sensitive carrier>

It became clear that the integrated physiologically active substance was released from the aggregate of the pH sensitive carrier at a neutral pH. Since the pH of blood or blood plasma is neutral, it was concerned that when administration of an aggregate of the pH sensitive carrier integrated with a physiologically active substance into a living body is considered, as the pH becomes neutral, the aggregate of the pH sensitive carrier immediately releases the physiologically active substance, and the effect of integration may be lost. Thus, the time for retaining the aggregate of the pH sensitive carrier at a neutral pH was investigated.

OVA was used, and the concentration was adjusted to 0.8 mg/mL. Regarding the dispersion liquid of the pH sensitive carrier, a dispersion liquid containing 1,000 nmol of DLPC and 1,600 nmol of deoxycholic acid in 1 mL was used. The total amount of the final solution was adjusted to 2 mL. The pH at the time of preparing the aggregate of the pH sensitive carrier was adjusted to 2.6 using 0.1 N hydrochloric acid, and when the pH was returned to be neutral, 0.1 N sodium hydroxide was used. Furthermore, measurement of the absorbance before and after a filtration treatment was carried out by a method similar to that of section 2. of Experiment 2.

At a neutral pH, the aggregate of the pH sensitive carrier tended to disappear with time (Fig. 14A). Specifically, a precipitate was recognized in the range of 0 min to 3 hours while a precipitate could not be recognized at 5 hours. It became clear that the aggregate of the pH sensitive carrier can maintain a precipitated state for about 3 hours at a neutral pH. Furthermore, with regard to the results of the change in absorbance, it was also verified that aggregates integrated with the protein remained from 0 min to 3 hours (Fig. 14B).

<Experiment 13. Pharmacokinetics of aggregate of pH sensitive carrier>

A pH sensitive carrier fluorescently modified with Rhodamine-PE, or an aggregate of a pH sensitive carrier fluorescently modified with Rhodamine-PE was intradermally injected to C57BL/6 mice, and the local kinetics in vivo was compared. Regarding the dispersion liquid of the pH sensitive carrier, a dispersion liquid containing 1,000 nmol of DLPC and 1,600 nmol of deoxycholic acid in 1 mL was used. The pH at the time of preparing the aggregate of the pH sensitive carrier was adjusted to 4 using 1 N sulfuric acid. The time for standing was set to 1 hour.

Fig. 15A shows the external appearance of the pH sensitive carrier fluorescently modified with Rhodamine-PE, and the external appearance of the aggregate of the pH sensitive carrier fluorescently modified with Rhodamine-PE. After being intradermally injected, the local kinetics after about 5 minutes was evaluated, and the pH sensitive carrier exhibited a wide distribution centered around the site where the carrier was intradermally injected (Figs. 15B and 15C). On the other hand, the aggregate of the pH sensitive carrier showed a linear distribution (Figs. 15D and 15E). For reproduction, the aggregate of the pH sensitive carrier was intradermally injected at a plurality of sites, and for all of the administrations, linear local kinetics was observed, and reproduction was confirmed (Fig. 15F). It was verified that the pH sensitive carrier and the aggregate of the pH sensitive carrier exhibit different local kinetics. It is speculated that since the aggregate of the pH sensitive carrier are very large particles, the aggregate exhibits local kinetics different from that of the pH sensitive carrier. Meanwhile, the aggregate of the pH sensitive carrier was formed into a suspension by pipetting before administration.

From the above results, since the pH sensitive carrier and the aggregate of the pH sensitive carrier exhibit different local kinetics, it became clear that the aggregate of the pH sensitive carrier exists in the living body without being dissociated and thus can be used in the living body.

The present application is based on Japanese Patent Application No. 2017-094952 filed on May 11, 2017, the disclosure of which is incorporated in its entirety by reference.

**Claims**

1. A complex comprising a physiologically active substance supported on an aggregate of a pH sensitive carrier, wherein the aggregate is formed by aggregating under acidic conditions.

2. The complex according to claim 1, wherein the acidic conditions are less than pH 6.4.

3. The complex according to claim 1 or 2, wherein the pH sensitive carrier contains at least one pH sensitive compound selected from the group consisting of deoxycholic acid, cholic acid, ursodeoxycholic acid, chenodeoxycholic acid, hyodeoxycholic acid, higher bile acid, glycodeoxycholic acid, glycyrrhizic acid, glycyrrhetinic acid, and salts thereof; and at least one amphiphilic substance selected from the group consisting of a phosphatidylcholine having 10 to 12 carbon atoms, a polyoxyethylene sorbitan monofatty acid ester having 12 to 18 carbon atoms, a sorbitan fatty acid ester having 16 to 18 carbon atoms, glycerol monooleate, glycerol dilaurate, glycerol distearate, glycerol dioleate, polyoxyethylene castor oil, and $\alpha$-tocopherol.

4. The complex according to any one of claims 1 to 3, wherein the physiologically active substance is a protein, a peptide, or a nucleic acid.

5. A pH sensitive composition comprising the complex according to any one of claims 1 to 4, and an aluminum compound.

6. A method for manufacturing the complex according to any one of claims 1 to 4, the method comprising:

preparing a pH sensitive carrier containing a pH sensitive compound and an amphiphilic substance;
mixing the pH sensitive carrier and a physiologically active substance to prepare a mixed liquid; and
adjusting the pH of the mixed liquid to a value less than 6.4.

FIG. 1(A)

FIG. 1(B)

FIG. 1(C)

FIG. 1(D)

FIG. 2(A)

FIG. 2(B)

EP 3 622 967 A1

FIG. 3(A)

FIG. 3(B)

FIG. 4(A)

FIG. 4(B)

FIG. 5(A)

| pH 7.4 | 6.8 | 6.4 | 5.9 | 3.8 | 2.6 | 2.2 | 2.1 | 1.9 |

FIG. 5(B)

FIG. 5(C)

FIG. 6

FIG. 7(A)

FIG. 7(B)

FIG. 8

FIG. 9(A)

FIG. 9(B)

Addition of Alum

FIG. 10(A)

FIG. 10(B)

FIG. 11

FIG. 12(A)

FIG. 12(B)

FIG. 13(A)

Immediately after pH adjustment

FIG. 13(B)

After standing for one day

FIG. 13(C)

FIG. 13(D)

FIG. 14(A)

FIG. 14(B)

FIG. 15(A)

FIG. 15(B)

FIG. 15(C)

FIG. 15(D)

FIG. 15(E)

FIG. 15(F)

## INTERNATIONAL SEARCH REPORT

| | International application No. |
|---|---|
| | PCT/JP2018/017982 |

| A. CLASSIFICATION OF SUBJECT MATTER |
|---|
| Int.Cl. A61K47/28(2006.01)i, A61K9/127(2006.01)i, A61K47/14(2006.01)i, A61K47/22(2006.01)i, A61K47/24(2006.01)i, A61K47/26(2006.01)i, A61K47/44(2017.01)i |
| According to International Patent Classification (IPC) or to both national classification and IPC |

| B. FIELDS SEARCHED |
|---|
| Minimum documentation searched (classification system followed by classification symbols) |
| Int.Cl. A61K47/28, A61K9/127, A61K47/14, A61K47/22, A61K47/24, A61K47/26, A61K47/44 |

| Documentation searched other than minimum documentation to the extent that such documents are included in the fields searched | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

| Electronic data base consulted during the international search (name of data base and, where practicable, search terms used) |
|---|
| JSTPlus/JMEDPlus/JST7580 (JDreamIII), CAplus/MEDLINE/EMBASE/BIOSIS (STN) |

| C. DOCUMENTS CONSIDERED TO BE RELEVANT |
|---|

| Category* | Citation of document, with indication, where appropriate, of the relevant passages | Relevant to claim No. |
|---|---|---|
| X | LONGO, G. S. et al., "pH-Controlled Nanoaggregation in Amphiphilic Polymer Co-networks", ACS Nano, 2013, vol. 7, pp. 2693-704, ISSN 1936-0851, abstract, page 2693, lower right column, etc. | 1, 4, 6 |
| X | WO 2015/079952 A1 (TERUMO CORP.) 04 June 2015, claims, paragraphs [0023], [0024], [0067], examples, etc. & US 2016/0271246 A1, claims, paragraphs [0070], [0071], [0116], examples & EP 3075393 A1 | 1-6 |
| X | WO 2013/180253 A1 (TERUMO CORP.) 05 December 2013, claims, examples, etc. & US 2013/0323320 A1, claims, examples & EP 2857043 A1 | 1-4, 6 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 May 2018 (30.05.2018) | 12 June 2018 (12.06.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013180253 A
- US 2013323320 A
- US 2016151497 A
- US 2018104343 A
- WO 2015079952 A
- US 2016271246 A
- WO 2016194685 A
- EP 3305320 A
- JP 2017094952 A

**Non-patent literature cited in the description**

- *Biomaterials,* 2008, vol. 29 (29), 4029-4036
- *Biomaterials,* 2013, vol. 34 (12), 3042-3052
- Liposomes. Nankodo Co., Ltd,
- Liposomes in Life Science. Springer-Verlag Tokyo, Inc,
- **K. KONO et al.** *Bioconjugate Chem.,* 2008, vol. 19, 1040-1048